# EUROPEAN PATENT APPLICATION

(11) **EP 3 017 825 A1**
(43) Date of publication of application: **11.05.2016**
(21) Application number: 14192043.9
(22) Date of filing: 06.11.2014
(51) Int. Cl.: A61K 38/17, C07K 16/24

(54) **An inhibitor of the RIPK1-dependent necrosis pathway and a pharmaceutical composition comprising the same**

(71) Applicant: Ludwig-Maximilians-Universität München, 80539 München (DE)
(72) Inventor: Anders, Hans-Joachim, 80993 München (DE); Mulay, Shrikant Ramesh, 80336 München (DE)
(74) Representative: Engelhard, Markus

(57) **Abstract**

The present invention relates to an inhibitor of the RIPK1-dependent necrosis pathway, to pharmaceutical compositions comprising the same, and to uses thereof.

SEQUENCE LISTING

<110>  Ludwig-Maximilians-Universität München

<120>  An inhibitor of the RIPK1-dependent necrosis pathway

<130>  U30565EP

<160>  12

<170>  PatentIn version 3.5

<210>  1
<211>  18
<212>  DNA
<213>  Artificial Sequence

<220>
<223>  forward primer Kim-1

<400>  1
tcagctcggg aatgcaca                                                    18


<210>  2
<211>  18
<212>  DNA
<213>  Artificial Sequence

<220>
<223>  reverse primer Kim-1

<400>  2
tggttgcctt ccgtgtct                                                    18


<210>  3
<211>  20
<212>  DNA
<213>  Artificial Sequence

<220>
<223>  forward primer pi-GST

<400>  3
cgcagcactg aatccgcacc                                                  20


<210>  4
<211>  21
<212>  DNA
<213>  Artificial Sequence

<220>
<223>  reverse primer pi-GST

<400>  4
acaccgccct cgaactggga a                                                21


<210>  5
<211>  22
<212>  DNA

<213>  Artificial Sequence

<220>
<223>  forward primer TNFR

<400>  5
gcaacagcac cgcagtagct ga                                                22


<210>  6
<211>  20
<212>  DNA
<213>  Artificial Sequence

<220>
<223>  reverse primer TNFR

<400>  6
gtgcgtccct tgcagccact                                                   20


<210>  7
<211>  22
<212>  DNA
<213>  Artificial Sequence

<220>
<223>  forward primer RIPK-1

<400>  7
gactgtgtac ccttacctcc ga                                                22


<210>  8
<211>  22
<212>  DNA
<213>  Artificial Sequence

<220>
<223>  reverse primer RIPK-1

<400>  8
cactgcgatc attctcgtcc tg                                                22


<210>  9
<211>  22
<212>  DNA
<213>  Artificial Sequence

<220>
<223>  forward primer RIPK-3

<400>  9
gaagacacgg cactccttgg ta                                                22


<210>  10
<211>  23
<212>  DNA
<213>  Artificial Sequence

<220>

<223>  reverse primer RIPK-3

<400>  10
cttgaggcag tagttcttgg tgg                                               23


<210>  11
<211>  22
<212>  DNA
<213>  Artificial Sequence

<220>
<223>  forward primer MLKL

<400>  11
ctgagggaac tgctggatag ag                                                22


<210>  12
<211>  22
<212>  DNA
<213>  Artificial Sequence

<220>
<223>  reverse primer MLKL

<400>  12
cgaggaaact ggagctgctg at                                                22

## Description

The present invention relates to an inhibitor of the RIPK1-dependent necrosis pathway, to pharmaceutical compositions comprising the same, and to the use thereof.

Crystals cause tissue injury in numerous disorders including gout, pseudogout, atherosclerosis, silicosis, asbestosis, rhabdomyolysis, crystal nephropathy, and nephro-/urolithiasis. Crystals are known to trigger immunopathology via the NLRP3 inflammasome, but the mode of direct crystal cytotoxicity is still unknown. Crystals are solid particles of different sizes and shapes that form from atoms, ions or molecules that assemble in an ordered three-dimensional pattern. Crystal deposits within the body are usually associated with injury, inflammation and tissue remodeling. The causality in this association can be both ways. Tissue injury serves as a nidus and promotes crystal adhesion and growth, e.g. urolithiasis forming at Randalls' plaques of the kidney papilla or calcifications forming at injured tendons, damaged cartilage, and atheromatous vascular lesions. In contrast, crystal formation itself causes tissue injury and inflammation, e.g. in gouty arthritis, silicosis or asbestosis of the lung, cholesterol crystals driving atherogenesis, and during oxalate, cystine or urate nephropathy.

Taking gout as an example, this is a disorder which is caused by elevated levels of uric acid in the blood leading to uric acid crystal formation, which crystals deposit in joints, tendons and surrounding tissues. The condition is characterized by recurrent attacks of acute inflammatory arthritis. Treatment is usually performed with non-steroidal anti-inflammatory drugs (NSAIDs), steroids, or colchicine. Once the acute inflammation has been alleviated, middle-term and long-term treatment is usually via lifestyle changes and dietary restrictions.

Urolithiasis is caused by the formation and aggregation of crystals from dietary minerals anywhere in the urinary system. Treatment is primarily symptomatic, aiming at a pain control using medications such a non-steroidal anti-inflammatory drugs or opioids. More severe cases may require surgical intervention or destruction of the aggregation of crystals using extra corporeal shock wave lithotripsy (ESWL).

The phenomenon of neutrophil extracellular trap (NET)-formation was first described as a measure of an organism to enforce antibacterial defense, in that neutrophils are one of the first lines of defense against invading bacteria. Upon activation, neutrophils release chromatin decorated with granular proteins. This chromatin structure binds gram-positive and -negative bacteria as well as fungi and represents an extracellular trap, hence the term neutrophil extracellular trap (NET). Formation of such neutrophil extracellular traps is, however, also a recognized driver of vascular injuries, thrombosis, septic organ failure, gout and atherosclerosis. Accordingly, NET-formation-associated diseases, such as sepsis, thrombosis, myocardial infarction and autoimmune vasculitis may be aggravated or at least accompanied by such neutrophil extracellular trap-formation.

Accordingly, there remains a need in the art for other forms of treatment of various crystallopathies as well as NET-formation-related diseases/disorders.

In a first aspect, the present invention relates to an inhibitor of the receptor-interacting serine/threonine-protein kinase 1 (RIPK1)-dependent necrosis pathway for use in a method of treatment of a crystallopathy or a neutrophil extracellular trap (NET)-formation-associated disease.

In one embodiment, the inhibitor of the RIPK1-dependent necrosis pathway inhibits said pathway by inhibiting any of the following:
- binding of TNFα to TNFα-receptor (TNFR),
- internalization of TNF receptor into a cell,
- activity or expression of receptor-interacting serine/threonine-protein kinase 1 (RIPK1),
- activity or expression of receptor-interacting serine/threonine-protein kinase 3 (RIPK3),
- activity or expression of mixed lineage kinase-domain-like protein (MLKL),
or any combination of the foregoing.

In one embodiment, said inhibitor is
- an anti-TNFα-compound selected from etanercept, infliximab, adalimumab, inhibitors of TNF-receptor-internalization such as R7050, and anti-TNFα-siRNA,
- a RIPK1-inhibitor selected from necrostatin 1, necrostatin 1s, and anti-RIPKl-siRNA,
- a RIPK3-inhibitor selected from anti-RIPK3 siRNA, or
- a MLKL-inhibitor, preferably selected from necrosulfonamide and anti-MLKL-sirRNA.

In one embodiment, said crystallopathy is a disease selected from gout, pseudogout, silicosis, asbestosis, oxalosis, e.g. nephrocalcinosis, urolithiasis or nephrolithiasis, gallstones, pancreatic duct stones, cystinosis, atherosclerosis, implant-associated joint damage(s) and crystal-induced rhabdomyolysis.

In one embodiment, said crystallopathy is selected from gout and oxalosis.

In one embodiment, said neutrophil extracellular trap (NET)-formation-associated disease is selected from sepsis, thrombosis, autoimmune vasculitis, atherosclerosis, and myocardial infarction.

In one embodiment, said NET-formulation-associated disease is selected from sepsis, thrombosis, atherosclerosis and myocardial infarction.

The present invention also relates to a pharmaceutical composition comprising the inhibitor, as defined above, and a pharmaceutical excipient, for use in a method of treatment of a crystallopathy or a neutrophil extracellular trap (NET)-formation-associated disease, wherein said inhibitor, said crystallopathy and said NET-formation-associated disease are as defined above.

In one embodiment, said treatment comprises administering a suitable amount of said inhibitor to a patient suffering from a crystallopathy or a NET-formation-associated disease.

The present invention also relates to a use of receptor-interacting serine/threonine-protein kinase 1 (RIPK1), receptor-interacting serine/threonine-protein kinase 3 (RIPK3) and/or of mixed lineage kinase domain-like protein in a screening method for identifying a compound useful for the treatment of a crystallopathy or a neutrophil extracellular trap (NET)-formation-associated disease.

In one embodiment, said screening method for identifying involves:
- providing a candidate compound suspected of or to be tested for having an inhibitory action on RIPK1, RIPK3 or MLKL, and providing a known inhibitor of RIPK1, RIPK3, or MLKL,
- determining the action of said candidate compound and of a known inhibitor of RIPK1, RIPK3 or MLKL, on RIPK1, RIPK3 or MLKL,
- comparing the determined action of said candidate compound with the determined action of said known inhibitor of RIPK1, RIPK3 or MLKL, and
- identifying said candidate compound as being useful for the treatment of a crystallopathy or NET-formation-associated disease, if the action of said candidate compound on RIPK1, RIPK3 or MLKL is the same as the action of said known inhibitor, or if the action of said candidate compound on RIPK1, RIPK3 or MLKL is not less than 50% of the action of said known inhibitor, preferably not less than 40%, more preferably not less than 30%, even more preferably not less than 20%, and most preferably not less than 10% of the action of said known inhibitor.

The present invention also relates to a method of treatment of a crystallopathy or a neutrophil extracellular trap (NET)-formation-associated disease, comprising administering a suitable amount of an inhibitor of the RIPK1-dependent necrosis pathway, said inhibitor being as defined above, or of a pharmaceutical composition, as defined above, to a patient suffering from a crystallopathy or a NET-formation-associated disease.

In one embodiment, said crystallopathy and said NET-formation-associated disease are as defined in any of claims 4-7.

In one embodiment, said suitable amount for treatment is an amount in a range of from 0.001 mg/kg body weight of said patient to 1 g/kg body weight of said patient.

In one embodiment, said patient is human.

The present inventors have surprisingly found that crystals typically appearing in crystallopathies trigger cell necrosis in a strictly RIPK1-dependent manner, leading to a necrotic cell death. Likewise, the inventors have found that neutrophil extracellular trap (NET)-formation depends on receptor-interacting protein kinase (RIPK)-1, RIPK-3 and MLKL. Consequently, the crystal cytotoxicity as well as NET-formation relies on the RIPK1-dependent necrosis pathway. This RIPK1-dependent necrosis pathway thus is responsible for necrosis to occur upon exposure to crystals. As used herein, the term "RIPK1-dependent necrosis pathway" is used synonymously with the term "necroptosis pathway" or "RIPK1-RIPK3-MLKL-dependent necroptosis pathway". According to the invention, it appears that elements of the RIPK1-RIPK3-MLKL-dependent necroptosis pathway are required for crystal cytotoxicity. For example, a lack of RIPK3 in mice protects these from oxalate-induced renal tubule necrosis and acute kidney injury. In additional in-vitro and in-vivo studies, an essential role of the TNFR1-RIPK1-activation was identified in that etanercept, i.e. the soluble fusion protein of the extracellular ligand binding domain of human tumor necrosis factor receptor 2 (TNFR2) (TNFR2/p75) joined to the Fc subunit of human IgG1-antibody was effective in abrogating tubule necrosis and acute kidney injury upon crystal exposure. The same abrogation of tubule necrosis and prevention of acute kidney injury upon crystal exposure was also achieved by treatment with inhibitors of RIPK1, such as nectrostatin-1 or inhibitors of MLKL, such as necrosulfonamide. Similarly, RIPK1-inhibitor necrostatin-1 and MLKL-inhibitor necrosulfonamide as well as a genetic deficiency in RIPK3 or MLKL also prevent NET-formation in human neutrophils. Consequently, inhibitors of the RIPK1-dependent necrosis pathway are useful therapeutic options for crystallopathies to limit crystal-induced tissue injury, and for NET-neutrophil extracellular trap (NET)-related disorders such as sepsis, thrombosis, myocardial infarction and autoimmune vasculitis, by interfering with the RIPK1-dependent necrosis pathway (= necroptosis pathway = RIPK1-RIPK3-MLKL-dependent necroptosis pathway).

As used herein, the term "RIPK1-dependent necrosis pathway" is used interchangeably with the term "necroptosis pathway" or "RIPK1/RIPK3/MLKL-dependent necrosis pathway" or "RIPK2/RIPK3/MLKL-dependent necroptosis pathway".

The term "inhibitor of the RIPK1-dependent necrosis pathway" refers to any compound that inhibits such pathway at any level. Preferably, it inhibits RIPK1 or RIPK3 or MLKL or the binding of external stimuli, such as TNFα, to any of the receptors TNFR (tumor necrosis factor receptor) or or the activation of other downstream signals involved in this pathway, such as TRIF (= TIR-domain-containing adaptor-inducing interferon-beta).

Inhibition may, for example, occur by preventing the binding of TNFα to TNFα-receptor or by inhibiting the internalization of TNF-receptor. It may also or alternatively occur by inhibiting or preventing the activity or expression of RIPK1 or RIPK3. Alternatively, it may also occur by preventing or inhibiting the activity or expression of MLKL. Other levels of inhibition are also envisaged by the present invention, as long as such inhibition essentially inhibits the RIPK1-dependent necrosis pathway. For example, expression of RIPK1, RIPK3 or of MLKL may be inhibited by appropriate small interfering RNA (siRNA). Activity of RIPK1 may be inhibited by various means, for example by necrostatin-1 (i.e. 5-((1H-indol-3-yl)methyl)-3-methyl-2-thioxoimidazolidin-2-one or by necrostatin-1s, a more stable variant of necrostatin-1 (i.e. 5-((7-chloro-1H-indol-3-yl)methyl)-3-methylimidazolidine-2,4-dione)). Inhibition of mixed-lineage kinase domain-like protein (MLKL) can be achieved by necrosulfonamide.

The term "crystallopathy", as used herein, refers to a disease or disorder that is caused by or associated with the formation and/or accumulation of crystals in tissue. Examples for crystallopathies are gout, pseudogout, silicosis, asbestosis, oxalosis, for example nephrocalcinosis, urolithiasis, nephrolithiasis. Further examples are cystinosis, atherosclerosis and implant-associated joint damage(s) and rhabdomyolysis.

The term "neutrophil extracellular trap (NET)-formation-associated disease" or "neutrophil extracellular trap-formation-associated disorder", as used herein, is meant to refer to a disease or disorder that is accompanied, aggravated or caused by neutrophil extracellular trap (NET)-formation. Examples for such NET-formation-associated disease or disorder are sepsis, thrombosis, autoimmune vasculitis, atherosclerosis and myocardial infarction.

The present invention also relates to the use of an inhibitor of the RIPK1-dependent necrosis pathway for the manufacture of a medicament in a method of treatment of a crystallopathy or a neutrophil extracellular trap (NET)-formation-associated disease or disorder, wherein said inhibitor, said crystallopathy, said NET-formation-associated disease and said method of treatment are as defined further above.

In one embodiment according to the present invention, the inhibitor of the RIPK1-dependent necrosis pathway is formulated in a pharmaceutical composition. It should be noted that the inhibitors of the RIPK1-dependent necrosis pathway may also be formulated in the form of a pharmaceutically acceptable salt thereof.

### PHARMACEUTICAL ACCEPTALBE SALTS

Examples of pharmaceutically acceptable addition salts include, without limitation, the nontoxic inorganic and organic acid addition salts such as the acetate derived from acetic acid, the aconate derived from aconitic acid, the ascorbate derived from ascorbic acid, the benzenesulfonate derived from benzensulfonic acid, the benzoate derived from benzoic acid, the cinnamate derived from cinnamic acid, the citrate derived from citric acid, the embonate derived from embonic acid, the enanthate derived from enanthic acid, the formate derived from formic acid, the fumarate derived from fumaric acid, the glutamate derived from glutamic acid, the glycolate derived from glycolic acid, the hydrochloride derived from hydrochloric acid, the hydrobromide derived from hydrobromic acid, the lactate derived from lactic acid, the maleate derived from maleic acid, the malonate derived from malonic acid, the mandelate derived from mandelic acid, the methanesulfonate derived from methane sulphonicacid, the naphthalene-2-sulphonate derived from naphthalene-2-sulphonic acid, the nitrate derived from nitric acid, the perchlorate derived from perchloric acid, the phosphate derived from phosphoric acid, the phthalate derived from phthalic acid, the salicylate derived from salicylic acid, the sorbate derived from sorbic acid, the stearate derived from stearic acid, the succinate derived from succinic acid, the sulphate derived from sulphuric acid, the tartrate derived from tartaric acid, the toluene-p-sulphonate derived from p-toluene sulphonic acid, and the like. Such salts may be formed by procedures well known and described in the art.

In another embodiment, the compounds of the invention are used in their respective free base form, where available, according to the present invention.

Metal salts of a chemical compound of the invention include alkali metal salts, such as the sodium salt of a chemical compound of the invention containing a carboxy group.

The chemical compounds of the invention may be provided in unsolvated or solvated forms together with a pharmaceutically acceptable solvent(s) such as water, ethanol, and the like. Solvated forms may also include hydrated forms such as the monohydrate, the dihydrate, the hemihydrate, the trihydrate, the tetrahydrate, and the like. In general, solvated forms are considered equivalent to unsolvated forms for the purposes of this invention.

### ADMINISTRATION AND FORMULATION

The production of medicaments containing the compounds of the invention, its active metabolites or isomers and salts according to the invention and their application can be performed according to well-known pharmaceutical methods.

While the compounds of the invention, useable according to the invention for use in therapy, may be administered in the form of the raw chemical compound, it is preferred to introduce the active ingredient, optionally in the form of a physiologically acceptable salt in a pharmaceutical composition together with one or more adjuvants, excipients, carriers, buffers, diluents, and/or other customary pharmaceutical auxiliaries. Such salts of the compounds of the invention may be anhydrous or solvated.

In a preferred embodiment, the invention provides medicaments comprising a compound useable according to the invention, or a pharmaceutically acceptable salt or derivative thereof, together with one or more pharmaceutically acceptable carriers therefor, and, optionally, other therapeutic and/or prophylactic ingredients. The carrier(s) must be "acceptable" in the sense of being compatible with the other ingredients of the formulation and not harmful to the recipient thereof.

A medicament of the invention may be those suitable for oral, rectal, bronchial, nasal, topical, buccal, sub-lingual, transdermal, vaginal or parenteral (including cutaneous, subcutaneous, intramuscular, intraperitoneal, intravenous, intraarterial, intracerebral, intraocular injection or infusion) administration, or those in a form suitable for administration by inhalation or insufflation, including powders and liquid aerosol administration, or by sustained release systems. Suitable examples of sustained release systems include semipermeable matrices of solid hydrophobic polymers containing the compound of the invention, which matrices may be in form of shaped articles, e.g. films or microcapsules.

The compounds useable according to the invention, together with a conventional adjuvant, carrier, or diluent, may thus be placed into the form of medicament and unit dosages thereof. Such forms include solids, and in particular tablets, filled capsules, powder and pellet forms, and liquids, in particular aqueous or non-aqueous solutions, suspensions, emulsions, elixirs, and capsules filled with the same, all for oral use, suppositories for rectal administration, and sterile injectable solutions for parenteral use. Such medicament and unit dosage forms thereof may comprise conventional ingredients in conventional proportions, with or without additional active compounds or principles, and such unit dosage forms may contain any suitable effective amount of the active ingredient commensurate with the intended daily dosage range to be employed.

The compounds useable according to the invention can be administered in a wide variety of oral and parenteral dosage forms. It will be obvious to those skilled in the art that the following dosage forms may comprise, as the active component, either a compound(s) useable according to the invention or a pharmaceutically acceptable salt of a compound(s) useable according to the invention.

For preparing a medicament from a compound useable according to the invention, pharmaceutically acceptable carriers can be either solid or liquid. Solid form preparations include powders, tablets, pills, capsules, cachets, suppositories, and dispersible granules. A solid carrier can be one or more substances which may also act as diluents, flavouring agents, solubilizers, lubricants, suspending agents, binders, preservatives, tablet disintegrating agents, or an encapsulating material.

In powders, the carrier is a finely divided solid which is in a mixture with the finely divided active component. In tablets, the active component is mixed with the carrier having the necessary binding capacity in suitable proportions and compacted in the shape and size desired. Suitable carriers are magnesium carbonate, magnesium stearate, talc, sugar, lactose, pectin, dextrin, starch, gelatin, tragacanth, methylcellulose, sodium carboxymethylcellulose, a low melting wax, cocoa butter, and the like. The term "preparation" is intended to include the formulation of the active compound with encapsulating material as carrier providing a capsule in which the active component, with or without carriers, is surrounded by a carrier, which is thus in association with it. Similarly, cachets and lozenges are included. Tablets, powders, capsules, pills, cachets, and lozenges can be used as solid forms suitable for oral administration.

For preparing suppositories, a low melting wax, such as a mixture of fatty acid glyceride or cocoa butter, is first melted and the active component is dispersed homogeneously therein, as by stirring. The molten homogenous mixture is then poured into convenient sized moulds, allowed to cool, and thereby to solidify. Compositions suitable for vaginal administration may be presented as pessaries, tampons, creams, gels, pastes, foams or sprays containing in addition to the active ingredient such carriers as are known in the art to be appropriate. Liquid preparations include solutions, suspensions, and emulsions, for example, water or water-propylene glycol solutions. For example, parenteral injection liquid preparations can be formulated as solutions in aqueous polyethylene glycol solution.

The chemical compounds according to the present invention may thus be formulated for parenteral administration (e.g. by injection, for example bolus injection or continuous infusion) and may be presented in unit dose form in ampoules, pre-filled syringes, small volume infusion or in multi-dose containers with an added preservative. The compositions may take such forms as suspensions, solutions, or emulsions in oily or aqueous vehicles, and may contain formulation agents such as suspending, stabilising and/or dispersing agents. Alternatively, the active ingredient may be in powder form, obtained by aseptic isolation of sterile solid or by lyophilization from solution, for constitution with a suitable vehicle, e.g. sterile, pyrogen-free water, before use.

Aqueous solutions suitable for oral use can be prepared by dissolving the active component in water and adding suitable colorants, flavours, stabilising and thickening agents, as desired. Aqueous suspensions suitable for oral use can be made by dispersing the finely divided active component in water with viscous material, such as natural or synthetic gums, resins, methylcellulose, sodium carboxymethylcellulose, or other well-known suspending agents.

Also included are solid form preparations which are intended to be converted, shortly before use, to liquid form preparations for oral administration. Such liquid forms include solutions, suspensions, and emulsions. These preparations may contain, in addition to the active component, colorants, flavours, stabilisers, buffers, artificial and natural sweeteners, dispersants, thickeners, solubilizing agents, and the like.

In one embodiment of the present invention, the medicament is applied topically or systemically or via a combination of the two routes.

For administration, the compounds of the present invention may, in one embodiment, be administered in a formulation containing 0,001% to 70% per weight of the compound, preferably between 0,01% to 70% per weight of the compound, even more preferred between 0,1% and 70% per weight of the compound. In one embodiment, a suitable amount of compound administered is in the range of 0.001 mg/kg body weight to 1 g/kg body weight of the patient.

Compositions suitable for administration also include lozenges comprising the active agent in a flavoured base, usually sucrose and acacia or tragacanth; pastilles comprising the active ingredient in an inert base such as gelatin and glycerol or sucrose and acacia; and mouthwashes comprising the active ingredient in a suitable liquid carrier.

Solutions or suspensions are applied directly to the nasal cavity by conventional means, for example with a dropper, pipette or spray. The compositions may be provided in single or multi-dose form. In the latter case of a dropper or pipette, this may be achieved by the patient administering an appropriate, predetermined volume of the solution or suspension. In the case of a spray, this may be achieved for example by means of a metering atomising spray pump.

Administration to the respiratory tract may also be achieved by means of an aerosol formulation in which the active ingredient is provided in a pressurised pack with a suitable propellant such as a chlorofluorocarbon (CFC) for example dichlorodifluoromethane, trichlorofluoromethane, or dichlorotetrafluoroethane, carbon dioxide, or other suitable gas. The aerosol may conveniently also contain a surfactant such as lecithin. The dose of drug may be controlled by provision of a metered valve.

Alternatively the active ingredients may be provided in the form of a dry powder, for example a powder mix of the compound in a suitable powder base such as lactose, starch, starch derivatives such as hydroxypropylmethyl cellulose and polyvinylpyrrolidone (PVP). Conveniently the powder carrier will form a gel in the nasal cavity. The powder composition may be presented in unit dose form for example in capsules or cartridges of, e.g., gelatin, or blister packs from which the powder may be administered by means of an inhaler.

In compositions intended for administration to the respiratory tract, including intranasal compositions, the compound will generally have a small particle size for example of the order of 5 microns or less. Such a particle size may be obtained by means known in the art, for example by micronization.

When desired, compositions adapted to give sustained release of the active ingredient may be employed.

The pharmaceutical preparations are preferably in unit dosage forms. In such form, the preparation is subdivided into unit doses containing appropriate quantities of the active component. The unit dosage form can be a packaged preparation, the package containing discrete quantities of preparation, such as packaged tablets, capsules, and powders in vials or ampoules. Also, the unit dosage form can be a capsule, tablet, cachet, or lozenge itself, or it can be the appropriate number of any of these in packaged form. Tablets or capsules for oral administration and liquids for intravenous administration and continuous infusion are preferred compositions.

Further details on techniques for formulation and administration may be found in the latest edition of Remington's Pharmaceutical Sciences (Maack Publishing Co. Easton, Pa.) and Remington: The science and practice of pharmacy", Lippincott Williams and Wilkins.

Appropriate formulations and ways of manufacturing them are, for example also disclosed in "Arzneiformenlehre, Paul Heinz List, Ein Lehrbuch für Pharmazeuten, Wissenschaftliche Verlagsgesellschaft Stuttgart, 4. Auflage, 1985", or "The theory and practice of industrial pharmacy" by Lachman et al., Varghese Publishing House, 1987", or "Modern Pharmaceutics", edited by James Swarbrick, 2. Edition".

The present invention also relates to a screening method for identifying a compound that is useful for the treatment of a crystallopathy or a NET-formation-associated disease or disorder. In embodiments of such screening method, a candidate compound which is suspected to be useful for the treatment of a crystallopathy or a NET-formation-associated disease or disorder, is tested for an inhibitory action on RIPK1, RIPK3 or MLKL, by determining the action of said candidate compound on RIPK1, RIPK3 or MLKL, and this action is compared with the action of a known inhibitor of RIPK1, RIPK3 or MLKL. Those candidate compounds which have the same action as a known inhibitor, such as necrostatin-1, necrostatin-1s, necrosulfonamide or etanercept, or the action of which is not less than 50% of the action of a known inhibitor, is identified as being useful for the treatment of a crystallopathy or NET-formation-associated disease. The term "is not less than 50% of the action of a known inhibitor" is meant to refer to a scenario wherein the actions of the candidate compound and of the known inhibitor are quantified and compared with each other. If the action of the candidate compound is less than 50% of the action of the known inhibitor, this will effectively mean that such candidate compound is not likely to be a useful candidate for the treatment of a crystallopathy or NET-formation-associated disease or disorder.

In the following, reference is made to the figures wherein
Figure 1 shows that crystals induce primary cell necrosis.,
Figure 2 shows that TNFRs and RIPK-1 mediate crystal cytotoxicity.
Figure 3 shows that a lack or suppression of RIPK1, RIPK3, and MLKL prevents crystal cytotoxicity.
Figure 4 shows that acute oxalate nephropathy involves tubular epithelial cell necrosis.
Figure 5 shows that oxalate nephropathy in C57BL/6 mice requires RIPK3 and MLKL.
Figure 6 shows RIPK1 inhibition and neutrophil recruitment.
Figure 7 shows that oxalate nephropathy in C57BL/6 mice requires TNFR1.
Figure 8 shows that etanercept, R-7050, and necrostatin-1 abrogate crystal nephropathy.
Figure 9 shows that the effect of CaOx crystals on MTCs resembles the effect of TNF-α.
Figure 10 shows the effect of Nec-1 (=nectrostatin 1) and ZVAD treatment on crystal induced cytotoxicity in human dermal fibroblasts, L929 cells and HK-2 cells.
Figure 11 shows the effect of Nec-1 treatment (Nec-1 = necrostatin-1) on neutrophil recruitment and inflammatory cytokine expression in air-pouches of mice after crystal injection.
Figure 12 shows the expression of TNFR-1 and TNFα in the kidneys of mice having acute oxalate nephropathy and TNF-α producing renal cells by identified by FACS.
Figure 13 shows human neutrophil studies in which human neutrophils were exposed to phorbol myristate acetate (PMA) in the presence of several inhibitors,
Figure 14 shows corresponding studies on mouse neutrophils,
Figure 15 shows sytox staining of human neutrophils after exposure to various stimuli and necrostatin-1 and necrosulfonamide,
Figure 16 shows quantitative analysis of western blots from RIPK-1, RIPK-3 and MLKL at different time points after PMA-stimulation of human neutrophils.
Figure 17 shows TEM images of human neutrophils after MSU stimulations in the presence of absence of Nec-1.
Figure 18 shows the effect of PMA stimulations on neutrophils lacking TNFR1 and TNFR2 (TNFR double knock-out (DKO)).

More specifically, reference is made to the following specific description, detailed discussion of the figures and examples which are given to illustrate, not to limit the present invention.

### Examples

### Example 1

### Material and Methods

### a) Methods for crystallopathy experiments

### Animal studies

C57BL/6N mice were procured from Charles River Laboratories (Sulzfeld, Germany). *Tnfr1-*/- and *Tnfr1*/*2-*/*-* mice were generously provided by V. Vielhauer, Germany. *Ripk3-*/*-* and *Mlkl-*/*-* mice were generously provided by A. Linkermann, Germany. Mice were housed in groups of five in filter top cages with unlimited access to food and water. Cages, nest lets, food and water were sterilized by autoclaving before use. Six-to-eight-week-old mice received a single intra-peritoneal injection of 100 mg/kg of sodium oxalate and 3% sodium oxalate in drinking water and kidneys were harvested after 24 hours. As a therapeutic strategy, mice received a single dose of either etanercept (10mg/kg *i.p.)* or necrostatin-1 (1.65mg/kg *i.p.*) or R-7050 (18mg/kg *i.p*.) before sodium oxalate injections. Blood and urine samples were collected before sacrifice by cervical dislocation and plasma and kidneys were harvested (ref). Kidneys were kept at -80°C for protein isolation and in RNA later solution at - 20°C for RNA isolation. One part of the kidney was also kept in formalin to be embedded in paraffin for histological analysis. All experimental procedures were approved by the local government authorities.

### Assessment of renal injury

Kidney sections of 2 µm were stained with periodic acid-Schiff (PAS) reagent. Tubular injury was scored by assessing the percentage of necrotic tubules and presence of tubular casts. Ly6B.2+ neutrophils were identified by immunostaining (clone 7/4, Serotec, Oxford, UK). Pizzolato's staining visualized calcium oxalate crystals and crystal deposit formation in the kidney was evaluated as follows: No deposits = 0 point; crystals in papillary tip = 1 point; crystals in cortical medullary junction = 2 points; crystals in cortex = 3 points. When crystals were observed in multiple areas, the points were combined. Plasma creatinine and BUN were measured using Urea FS kit (DiaSys Diagnostic Systems, Holzheim, Germany) according to the manufacturer's protocol. Neutrophil infiltrates were counted in 15 high power fields (hpf) per section. A cell death detection (TUNEL) kit (Roche, Mannheim, Germany) was used to quantify dead cells. All assessments were performed in a blind manner.

### Electron Microscopy (EM)

*Quick-Freeze Deep-Etch Electron Microscopy.* 1.5 mm bread slices of non-fixed kidney or kidney sections fi nxed overnight in 2% glutaraldehyde in 100 mM NaCl, 30 mM Hepes, 2 mM CaCl2, pH 7.2 (NaHCa) were used after rinsing in NaHCa. Tissues were then cooled to 4° K with liquid helium,mounted in a Balzers 400 vacuum evaporator fractured and etched for 2.5 minutes at minus 104° C and rotary replicated with ~ 3 nm platinum and viewed with on JEOL 1400 microscope with attached AMT digital camera.

### RNA preparation and real-time quantitative -PCR

Total RNA was isolated from kidneys using a Qiagen RNA extraction kit (Qiagen Germany) following the manufacturer's instructions. After quantification RNA quality was assessed using agarose gels. From isolated RNA, cDNA was prepared using reverse transcriptase (Superscript II) (Invitrogen, CA, USA). Real time RT-PCR was performed using SYBRGreen PCR master mix and was analyzed with a Light Cycler 480 (Roche, Germany). All gene expression values were normalized using 18s RNA as a house keeping gene. All primers used for amplification were from Metabion (Martinsried, Germany). The expression of KIM-1, π-GST, TNFR1, RIPK-1, RIPK-3 and MLKL,
Kim-1: TCAGCTCGGGAATGCACA (For) (SEQ ID NO: 1),
   TGGTTGCCTTCCGTGTCT (Rev) (SEQ ID NO: 2);
π-GST: CGCAGCACTGAATCCGCACC (For) (SEQ ID NO: 3);
   ACACCGCCCTCGAACTGGGAA (Rev) (SEQ ID NO: 4);
TNFR-1: GCAACAGCACCGCAGTAGCTGA (For) (SEQ ID NO: 5),
   GTGCGTCCCTTGCAGCCACT (Rev) (SEQ ID NO: 6);
RIPK-1: GACTGTGTACCCTTACCTCCGA (For) (SEQ ID NO: 7);
   CACTGCGATCATTCTCGTCCTG (Rev) (SEQ ID NO: 8);
RIPK-3: GAAGACACGGCACTCCTTGGTA (For) (SEQ ID NO: 9);
   CTTGAGGCAGTAGTTCTTGGTGG (Rev) (SEQ ID NO: 10);
MLKL: CTGAGGGAACTGCTGGATAGAG (For) (SEQ ID NO: 11);
   CGAGGAAACTGGAGCTGCTGAT (Rev) (SEQ ID NO: 12);

### Cell culture studies

Mouse tubular cell (MTCs), Human dermal fibroblasts (HDFs) and L929 cells were maintained in DMEM/F12 (GIBCO, Invitrogen, CA, USA) containing 10% fetal calf serum (FCS), 1% penicillin-streptomycin (PS). Human Kidney cells (HK-2) were maintained in RPMI (GIBCO, Invitrogen, CA, USA) containing 10% FCS, 1% PS. Cells were stimulated for 3 to 24 hours with different doses of crystals of CaOx (Alfa aesar, Germany), MSU (Invivogen, Germany), CPPD (Invivogen, Germany) and Cystine (Sigma Aldrich, Germany) in different experiments. Cells were pre-treated with Nec-1 (50 or 100µM) or zVAD (10 µM) or combination 30 min before crystals stimulations whenever required. Cell-free supernatants were analysed for cytokine secretion by ELISA (BD Pharmingen, Heidelberg, Germany). Primary tubular epithelial cells (pTECs) were isolated from the kidneys and were maintained in DMEM/F12 containing 10% fetal calf serum, 1% penicillin-streptomycin, 125 ng/ml prostaglandin E1 (Calbiochem, Germany), 25 ng/ml EGF, 1.8 µg/ml 1-thyroxine, 3.38 ng/ml hydrocortisone and 2.5 mg/ml of insulin-transferrin-sodium selenite supplement (I-T-SS) (all from Sigma Aldrich, Germany unless mentioned). Cells were grown to confluence before use in experiments. All the cells were cultured in an incubator at 37°C, 5%CO₂. CellTiter 96® Non-Radioactive Cell Proliferation Assay (MTT) Kit (Promega, Germany) was used to evaluate cell survival after 24 hours following the manufacturer's instructions. Results were expressed as percentage of the vehicle control.

### Protein isolations and Immunoblots

Protein from kidney tissues as well as cells were extracted with lysis buffer (Tris-HCl 50 mM, NaCl 150 mM, sodium orthovanadate 100 µM, sodium deoxycholate 0.5%, NP40 4%, Triton X-100 2%, EDTA 5 mM, sucrose 300 mM, and Roche protease inhibitors). Total cellular extracts were prepared in protein lysis buffer (Tris 10 mM, NaCl 10 mM, ethylenediaminetetraacetic acid 10 mM, HEPES 1 mol/l, glycerol 20%, MgCl2 1 mol/l, dithiothreitol 1 mol/l, Triton 10% sodium fluoride 1 mol/l, and Roche protease inhibitors). After determination of protein concentrations, 50 µg of the kidney protein or 10 µg of protein extracted from cells was mixed with 5 × sodium dodecyl sulfate loading buffer (100 mmol/l Tris-HCl, 4% sodium dodecyl sulfate, 20% glycerol, and 0.2% bromophenol blue) for western blot analysis. Samples were heated at 95 °C for 5 min. Proteins were separated by sodium dodecyl sulfate-polyacrylamide gel electrophoresis and then transferred to a polyvinylidene difluoride membrane. Nonspecific binding to the membrane was blocked for 1 h at room temperature with 5% bovine serum albumin in tris-buffered saline buffer (20 mmol/l Tris-HCl, 150 mmol/l NaCl, and 0.1% Tween 20). The membranes were then incubated overnight at 4°C with the following primary antibodies: TNFR1, RIPK1, RIPK3 (all Abcam, UK) and β-actin (Cell signalling, MA, USA), followed by incubation with secondary antibody anti-biotin or anti-rabbit IgG labeled with HRP. Immunostained bands were detected using a chemiluminescence kit (ECL kit, GE Healthcare, UK).

### Multi-parameter classification of cell death by flow cytometry

Cell death was induced in MTCs by irradiation with ultraviolet light type B (UVB) at 1.5 mJ/cm²/s or by treatment with calcium oxalate crystals. Cell death was characterized by analyzing six cytofluorometric parameters (size, granularity, PS exposure, plasma membrane integrity, mitochondrial membrane potential and DNA content) in a single tube-measurement. This method detects 8 phenotypically different subpopulations during the development of cell death in vitro. Briefly, harvested cells were incubated for 30 min at room temperature with 400 µl of freshly prepared 4-color staining solution (1.8 µg/ml AxA5-FITC, 100 ng/ml PI, 10 nM DiIC1(5), 1 ng/ml Hoechst 33342) in Ringer's solution and subsequently analyzed. Flow cytometry was performed with a Gallios cytofluorometer (Beckman Coulter, Fullerton, CA, USA). Excitation of FITC and PI was at 488 nm, the FITC fluorescence was detected with the FL1 sensor (525/38 nm BP), the PI fluorescence with the FL3 sensor (620/30 nm BP), the DiIC1(5) fluorescence was excited at 638 nm and detected with the FL6 sensor (675/20 nm BP), and the Hoechst 33342 fluorescence was excited at 405 nm and detected with the FL9 sensor (430/40 nm BP). Electronic compensation was applied to reduce bleed-through fluorescence. Data analysis was performed with Kaluza software version 2.0 (Beckman Coulter, Fullerton, CA, USA). Cells were classified according to their location in the forward scatter (FSc; size) vs. side scatter (SSc; granularity) dot plot and their staining patterns in the FL1 vs. FL3 and FL6 vs FL9 dot plots as described elsewhere (Munoz et. al. Autoimmunity (2013) 46:336-41).

### Mouse peritonitis model

Peritonitis was induced by injection of 1 mg of crystals in 0.5-ml sterile PBS. Mice received a single injection of either Nec-1 (1.65mg/kg) or vehicle half an hour before crystals injections. After 6 h, mice were killed and peritoneal cavities were washed with 5 ml of PBS. The lavage fluids were analyzed for PMN recruitment by FACS using the neutrophil marker Ly-6G (BD Biosciences, Germany).

### Air pouch experiments

Animal studies were conducted according to guidelines determined by the Law of the Ministry of Healthcare of Ukraine, No. 281 from November 1, 2011 for the care and use of laboratory animals and were approved by Ethics Council of the Danylo Halytsky Lviv National Medical University. Murine air pouches were generated using standard protocols in groups of 6 weeks old Balb/c mice (n=5). Subsequently, 2.5 mg crystals in PBS, 2.5 mg crystals plus 1.65 mg/kg necrostatin were injected into the air pouches. Crystals of CaOx, MSU, CPPD (calcium pyrophosphate dehydrate) and Cystine were used in each independent experiment. After 24hrs the pouch fluid was harvested, and neutrophils in the air lavage were analyzed by FACS.

### Mouse cremaster muscle IR injury assay

Cremaster muscle was prepared for experiment as originally described by Baez with minor modifications. Briefly, mice were anesthetized using intraperitoneal injection of ketamine/xylazine mixture. The left femoral artery was cannulated for administration of microspheres and drugs. The right cremaster muscle was exposed through a ventral incision of the scrotum. Epididymis and testicle were detached from the cremaster muscle and placed into the abdominal cavity. Throughout experimental procedure the muscle was superfused with warm-buffered saline.

Olympus BX 50 upright microscope (Olympus Microscopy, Germany) equipped for stroboscopic fluorescence epi-illumination microscopy was used for in vivo microsopy. For measurement of centerline blood flow velocity, green fluorescent microspheres (2 µm diameter, Molecular Probes, Leiden, The Netherlands) were injected via the femoral artery catheter, and their passage through the vessels of interest was recorded using the FITC filter cube under appropriate stroboscopic illumination (exposure, 1 ms; cycle time, 10 ms; 1=488 nm), integrating video images for sufficient time (>80 ms) to allow for the recording of several images of the same bead on one frame. Beads that were flowing freely along the centerline of the vessels were used to determine blood flow velocity. For off-line analysis of parameters describing the sequential steps of leukocyte extravasation, we used the Cap-Image image analysis software (Dr. Zeintl, Heidelberg, Germany).

Animals were treated with necrostatin-1 (1.65 mg/kg *i.p.)* or vehicle 30 min prior to the experiment. For the analysis of postischemic leukocyte responses, three postcapillary vessel segments in a central area of the spread out cremaster muscle were randomly chosen. Ischemia was induced by clamping all supplying vessels at the base of the cremaster muscle using a vascular clamp (Martin, Tuttlingen, Germany). After 30 min of ischemia, the vascular clamp was removed and reperfusion was restored for 160 min. Measurements were repeated at 60 and 120 min after onset of reperfusion. Subsequently, FITC dextran was infused *i.a.* for the analysis of microvascular permeability.

Analysis of microvascular permeability was performed as described previously. Briefly, the macromolecule FITC-dextran (5 mg in 0.1 ml saline, Mr 150,000, Sigma-Aldrich) was infused *i.a.* after determination of centerline blood flow velocity. Mean gray values of fluorescence intensity were measured by digital image analysis (TILLvisION 4.0, TILL Photonics) in six randomly selected ROIs (50x50 µm²), localized 50 µm distant from the postcapillary venule under investigation. The average of mean gray values was calculated.

### Statistical analysis

Data are presented as mean ± SEM. A comparison of groups was performed using students t test or ANOVA with post-hoc Bonferroni's correction was used for multiple comparisons. A value of p < 0.05 was considered to indicate statistical significance.

### b) methods for NET-formation-associated disorders

Neutrophils were isolated by from healthy volunteers or mice using standard Ficoll-Paque procedures as described. 0.5x106 or 5x106 neutrophils were suspended in 200 to 500µl RPMI and seeded into 8 well µ slides or 12 plates (Ibidi, Martinsried, Germany) to settle for 30 min in a 5% carbon dioxide atmosphere at 37°C. The human neutrophils were stimulated with different doses of necrostatin-1 (Nec-1, Enzo Life Sciences, Lörrach, Germany), Nec-1s (Biovision, Milpitas, CA) or necrosulfonamide (NSA, Millipore, Schwalbach, Germany) for 30 min. Neutrophils were further incubated with 100ng/ml LPS (Invivogen E. coli 0111:B4, Toulouse, France), 200nM TNF-α (Immunotools, Friesoythe, Germany), 25nM phorbol 12-myristate 13-acetate (PMA, Sigma-Aldrich, Steinheim, Germany) or 20pg/cell monosodium urate (MSU) crystals (Invivogen, Toulouse, France) for 2h.

Neutrophils were stimulated for 2h before stained with 0.1 µM Sytox green dye (Life Technologies, Eugene, Oregon) for 10 min for the presence of extracellular DNA. DNA release was determined by adding 50µl of PicoGreen dsDNA assay kit reagent (Life Technologies, Eugene, OR) (1:250 in TE buffer) to 50µl of cell culture supernatant and fluorescence was read after 5 min with an excitation wavelength of 485nm and emission wavelength of 535 nm. For confocal microscope imaging the cells were fixed using 4% paraformaldehyde and stained with anti-histone IgG and TO-PRO®-3 Iodide (Life Technologies). For electron microscopy cells were fixed in 2.0% paraformaldehyde/ 2.0% glutaraldehyde, in 0.1M sodium phosphate buffer, pH 7.4 for 24h, followed by 3 washes x15 min in 0.1m sodium phosphate buffer, pH 7.4 and distilled water. Again cells were post-fixed for TEM, in 2% OsO4 for 1h, dehydrated in graded ethanol and embedded in epon. Ultrathin sections, stained with uranyl acetate and lead citrate, were observed in a Zeiss Libra 120 electron microscope.

Cell proteins were separated by using 10% SDS-PAGE at constant voltage of 150V in standard running buffer until the gel front elute completely in Biorad gel electrophoresis system and wet-transferred to PVDF membrane and detected using antibodies for receptor-interacting protein kinase (RIPK)1 (BD Bioscience, San Diego, CA), RIPK3 (Abcam, Cambridge, UK), human-specific phospho-MLKL (Millipore, Billerica, MA), and β-actin (Cell Signaling, Leiden, The Netherlands).

Animal studies were approved by Ethics Council of the Danylo Halytsky Lviv National Medical University. Murine air pouches were generated using standard protocols in groups of 6 week old female Balb/c mice (n=5). Subsequently, 2.5 mg MSU crystals in PBS, PBS only, 2.5 mg MSU plus 1.65 mg/kg necrostatin-1 or necrostatin-1 alone were injected into the air pouches. 24h later the pouch fluid was harvested, and the DNA in the air lavage was analyzed by PicoGreen according to the manufacturer's instructions.

Data were calculated using ANOVA and Bonferroni correction where appropriate and expressed as means± standard error of the mean.

### Example 2

### Crystallopathy

### Crystals cause primary cell necrosis in a TNFR-dependent but caspase-independent manner

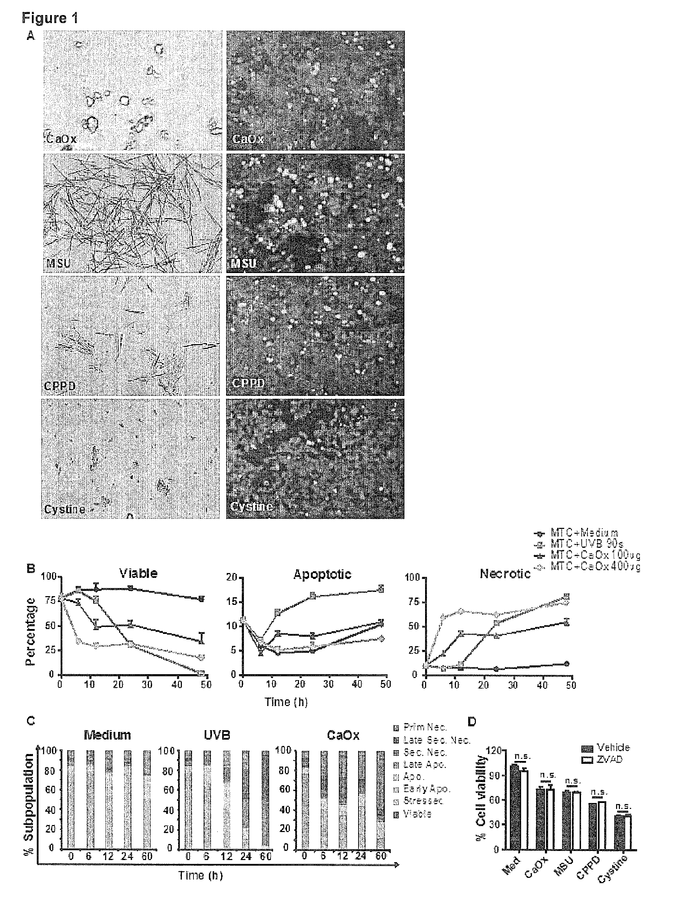

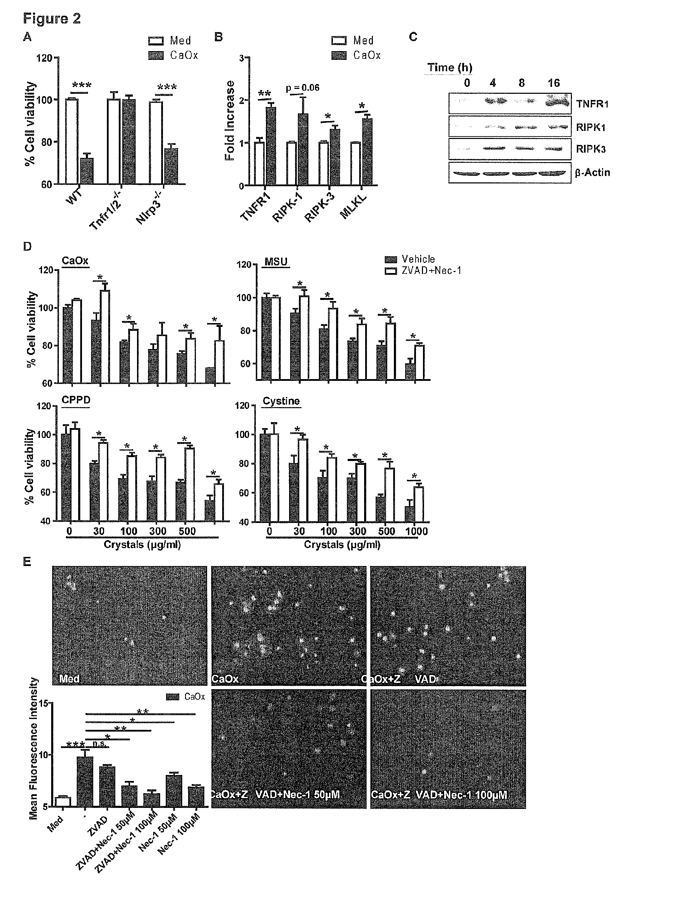

To address the question which mode the crystal-induced cell death adopts, the cytoxic effects of CaOx, MSU, CPPD, and cystine crystals on tubular epithelial cells in vitro were studied, and SSC, FSC, Hoechst 33342, annexin V, DiLC, and PI flow cytometry were used to better characterize the mode of CaOx- induced cell death (Figure 1A). CaOx crystals induced mainly primary necrosis and not apoptosis (Figure 1B and 1C), the latter being consistent with pan-caspase inhibitor ZVAD-FMK not affecting cytotoxicity by any of the aforementioned crystals (Figure 1D). Lack of NLRP3 did not affect CaOx crystal cytotoxicity (Figure 2A), which, together with being a caspase-independent mode of cell death, excluded NLRP3-caspase-1/11-dependent pyroptosis. In contrast, TNFR1/2-deficiency entirely abrogated CaOx crystal-induced cytotoxicity (Figure 2A). The inventors concluded that crystal cytotoxicity does not involve apoptosis or pyroptosis but largely represents primary cell necrosis involving TNFR signalling.

### Downstream of TNFRs crystal toxicity involves the RIPK1-RIPK3-MLKL necroptosis pathway

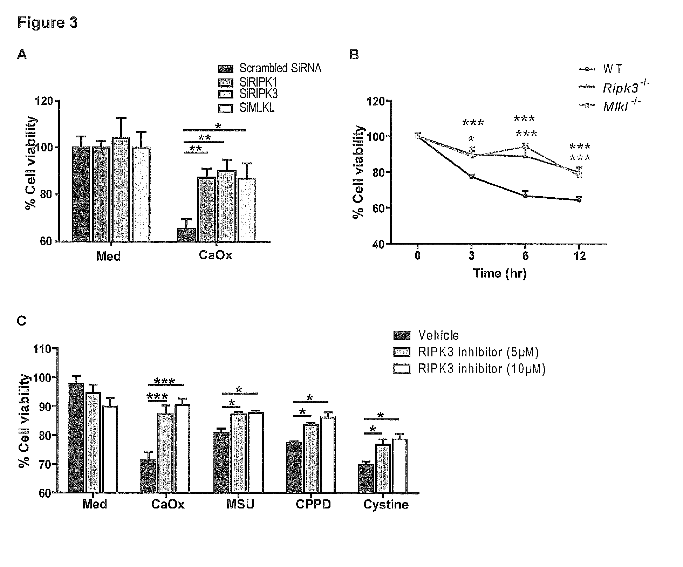

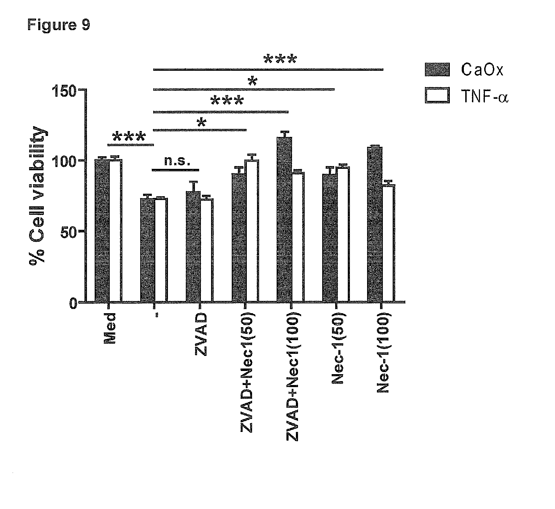

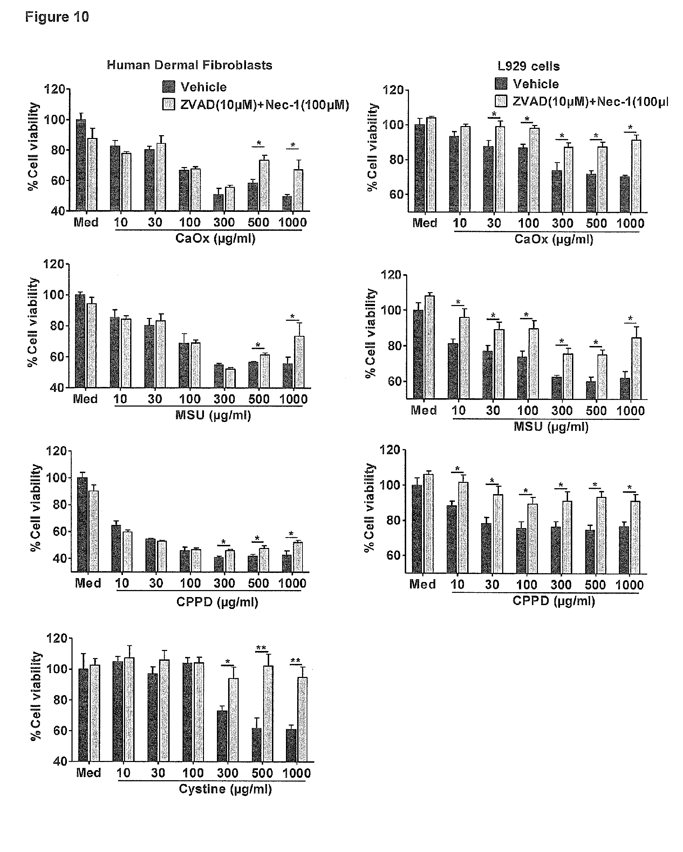

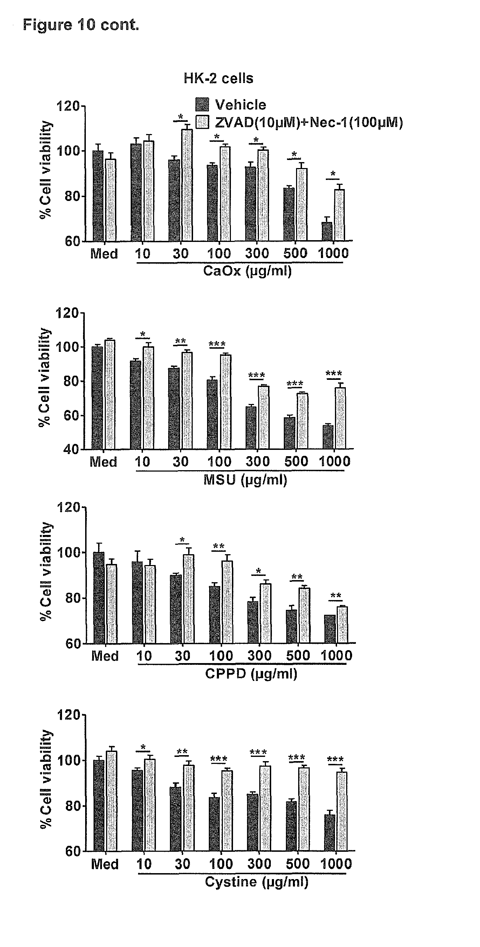

TNFR1/2 are members of the death receptor family that trigger cell death either via caspase-dependent apoptosis or RIPK1/RIPK3/MLKL-dependent necroptosis. Having excluded apoptosis, the inventors now focussed on necroptosis also because all elements of the pathway were induced in tubular epithelial cells upon crystal challenge at the mRNA and the protein level (Figures 2B and 2C). The small molecule RIPK1 antagonist necrostatin-1 (Nec-1) abrogated crystal-induced tubular epithelial cell death in a dose-dependent manner with and w/o ZVAD (Figure 2D). When crystals were replaced by recombinant TNF-α as a stimulus, necrostatin-1 had the same effect on cell viability (see also figure 9). Necrostatin-1 also suppressed crystal cytotoxicity in L929 cells, primary dermal fibroblasts, and HK-2 cells (see also figure 10). This preventive effect of necrostatin-1 on crystal cytotoxicity was confirmed by fluorescence imaging using the cell death marker Sytox that can enter cells only upon plasma membrane disruption (Figure 2E). Knock down of either RIPK1, RIPK3 or MLKL with specific siRNA partially abrogated CaOx-induced cytotoxicity in tubular epithelial cells (Figure 3A). Furthermore, primary tubular epithelial cells isolated from either *Ripk3-* or *Mlkl*-deficient mice were protected from CaOx-induced cell death (Figure 3B). Pretreatment of tubular cells with RIPK3 inhibitor also abrogated crystal induced cytotxicity (Figure 3C). Together these data imply that crystal-induced primary necrosis involves the TNFR-RIPK1-RIPK3-MLKL necroptosis pathway.

### Ripk3- and Mlkl-deficient mice are protected from crystal-induced kidney tubule necrosis

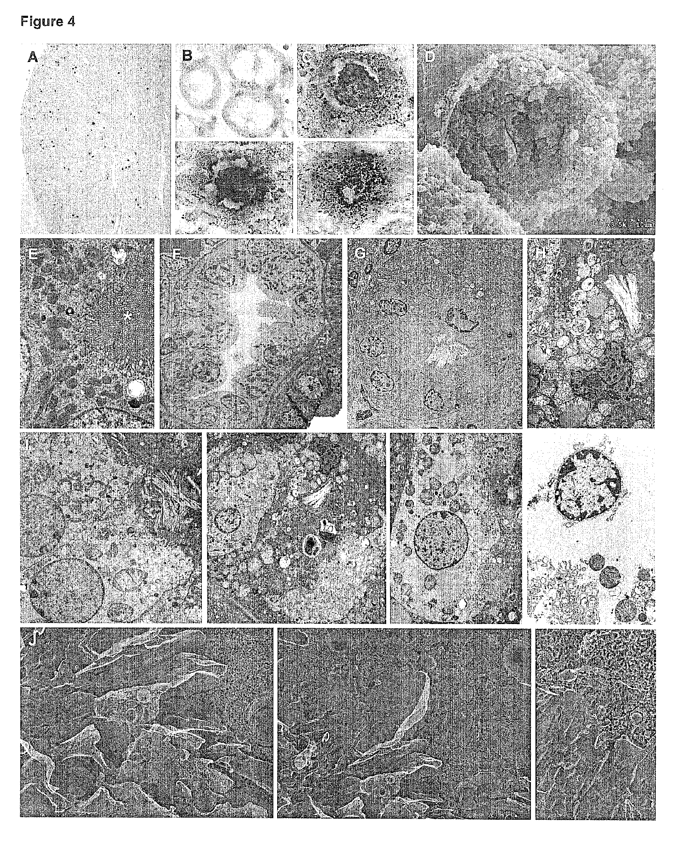

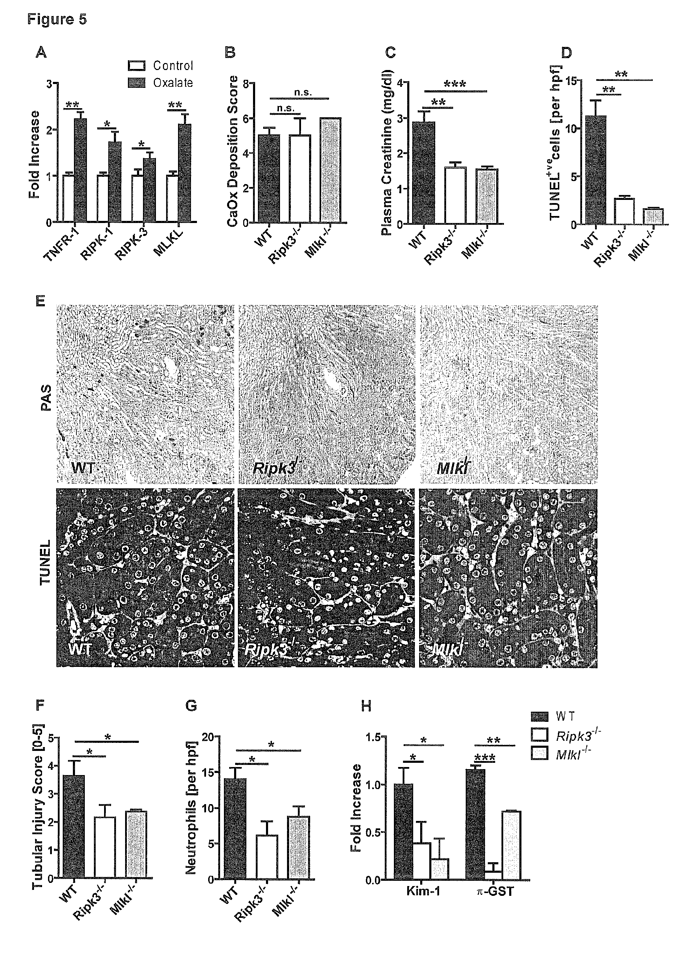

Crystal nephropathy (CN) is one example of crystal-induced tissue injury and organ failure. Oxalate exposure to mice induces oxalate nephropathy, a model that mimics CN in humans, including an increase of serum creatinine and blood urea nitrogen (BUN). Acute oxalate exposure leads to CaOx crystal deposition in the mouse kidney. At the structural level CaOx crystal plugs form within proximal and distal tubules (Figure 4A-D). CaOx crystals are also found within tubules and the interstitial compartment (Figure 4C). Transmission electron microscopy shows luminal crystal plugs as well as smaller-sized crystals within tubular epithelial cells (Figure 4E-G). Such tubular epithelial cells often show ultrastructural signs of necrosis such as mitochondrial balloning and translucence, chromatin irregularities, outer membrane disruption, and cytoplasma and organelle release into the extracellular space (Figure 4H). Quick-freeze deep-etch electron microscopy better illustrates how crystals get in touch with cell cytoplasma and organelles upon rupture of outer cell membranes as shown in the three parts of figure 4I. Thus, crystal formation within the kidney is associated with tubular epithelial cell necrosis in a temporal and spatial manner, a phenotype that is associated with induction of renal TNFR1, RIPK1, RIPK3, and MLKL mRNA expression (Figure 5A). The inventors validated the in vivo contribution of this pathway by inducing oxalate nephropathy in wild-type and *Ripk3-* and *Mlkl*-deficient mice. Oxalate exposure induced identical CaOx crystal deposits in both mouse strains (Figure 5B) but all functional and structural parameters of acute CN were significantly reduced in *Ripk3-* or *Mlkl*-deficient mice (Figures 5B-H). These include serum creatinine levels, markers of tubule necrosis, and neutrophil recruitment. Together, CaOx crystal-induced tubule necrosis and CN involves RIPK3-MLKL signalling.

### RIPK1 signalling does not mediate crystal-induced neutrophil recruitment

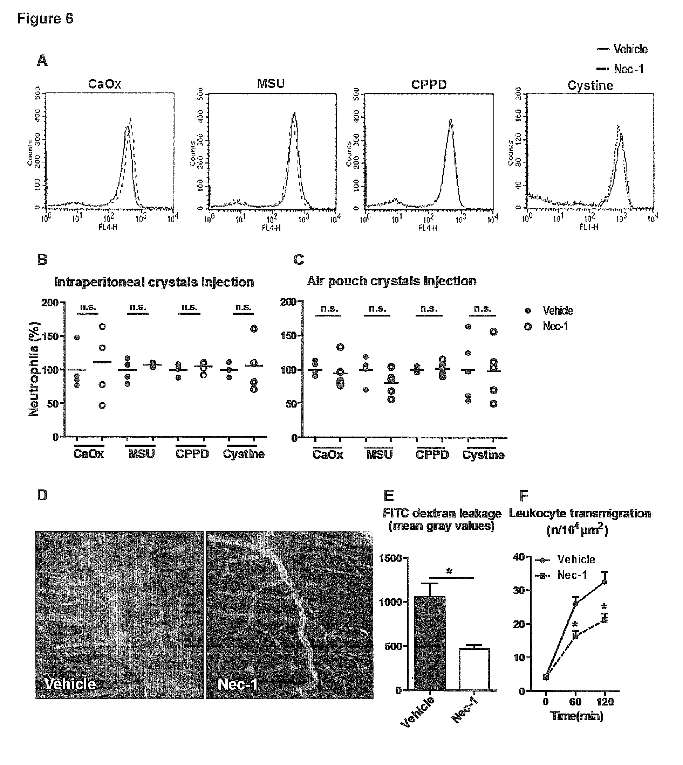

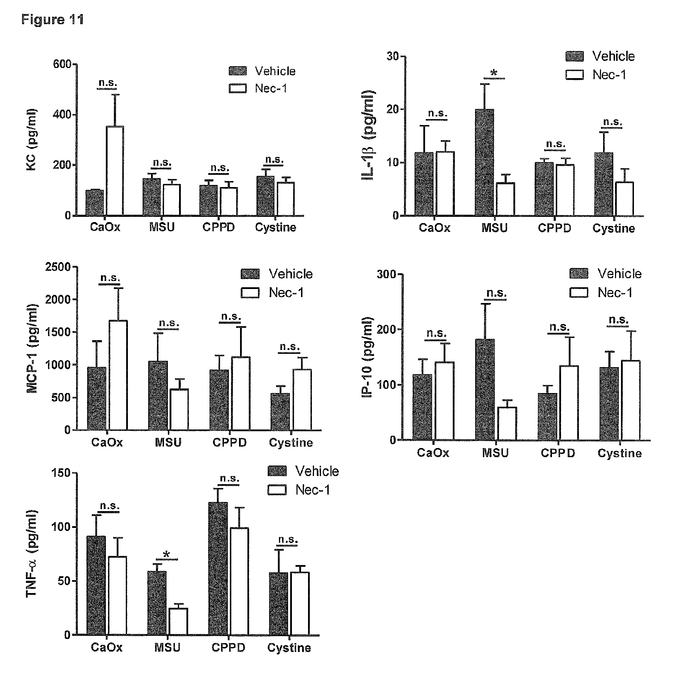

To exclude that tubule protection in *Ripk3-* and *Mlkl*-deficient mice is a secondary effect because the RIPK1-RIPK3-MLKL pathway might be involved in neutrophil recruitment, several in vitro and in vivo experiments were performed. Intraperitoneal injection of various crystals induced neutrophil recruitment into the peritoneal cavity as a marker of crystal-induced peritonitis but this was not affectd by necrostatin-1 (Figure 6A and 6B). Crystal injection into airpouches at the back of mice with and without systemic necrostatin-1 treatment gave identical results (Figure 6C). None of the aforementioned crystals induced release of cytokine or chemokine expression in air pouch fluid was affected by Nec-1 treatment (see figure 11). In contrast, necrostatin-1 significantly reduced microvascular permeability and leukocyte extravasation from postischemic cremaster muscles as captured by in vivo microscopy (Figure 6D-F). It can be concluded that, RIPK1-mediated tissue injury involves neutrophil recruitment as a secondary event that itself is independent of RIPK1.

### TNFR1 signaling serves as a trigger for tubular cell necroptosis in crystal nephropathy

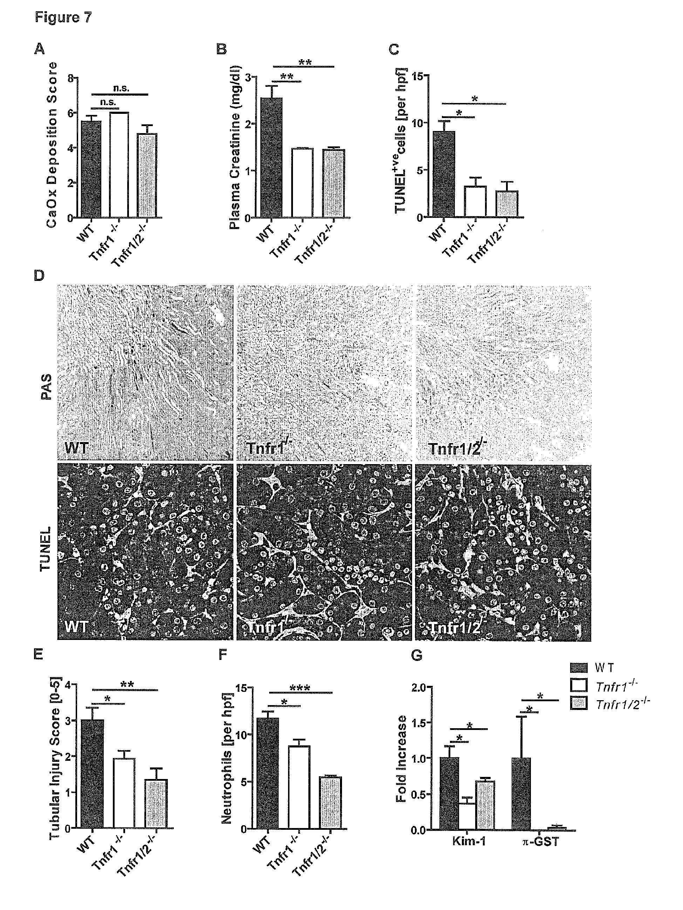

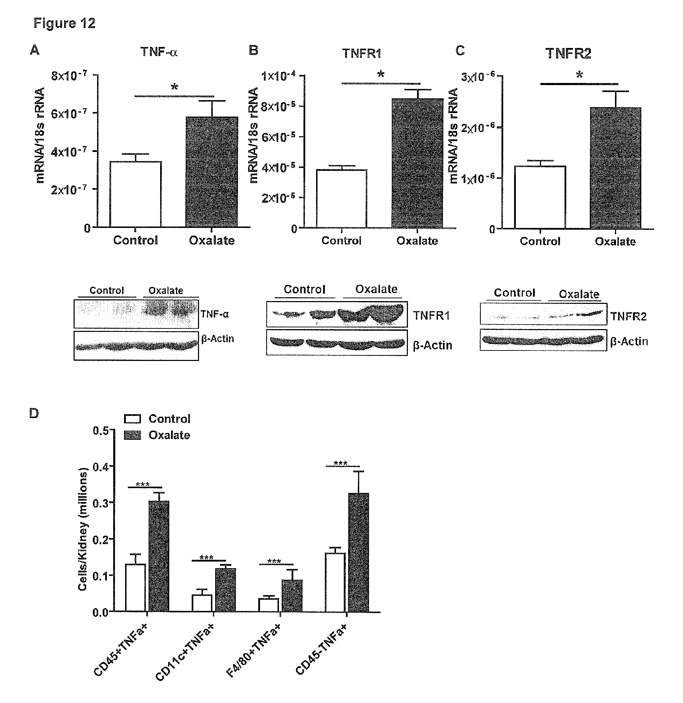

When crystals need TNFRs to induce tubular epithelial cell death in vitro then tubule injury and kidney dysfunction during CN may also depend on the presence of these receptors. Renal mRNA and protein levels of TNFα and both TNFRs were found to be induced in CaOx nephropathy (figure 12A-C). Flow cytometry revealed that TNFα was expressed by intrarenal mononuclear phagocytes but also by non-immune cells (figure 12D). To test for the functional contribution of TNFR signaling CN was induced in *Tnfr1*-deficient and *Tnfr1*/*2* double-deficient mice. Oxalate exposure resulted in identical CaOx crystal deposits in all mouse strains but all functional and structural parameters of acute CN were significantly reduced in *Tnfr1*-deficient mice (Figure 7). These include serum creatinine levels, blood urea introgen (BUN), tubule necrosis and neutrophil recruitment. Concomitant deficiency in TNFR2 did not have additive effects on this phenotype, suggesting that TNFR1 mainly accounts for this effect. Thus, TNFR1 signalling an important inducer of RIPK3-dependent cell death contributes to CN.

### TNF blockade with etanercept, TNFR blockade with R-7050 or necroptosis blockade with necrostatin-1 all abrogate crystal nephropathy

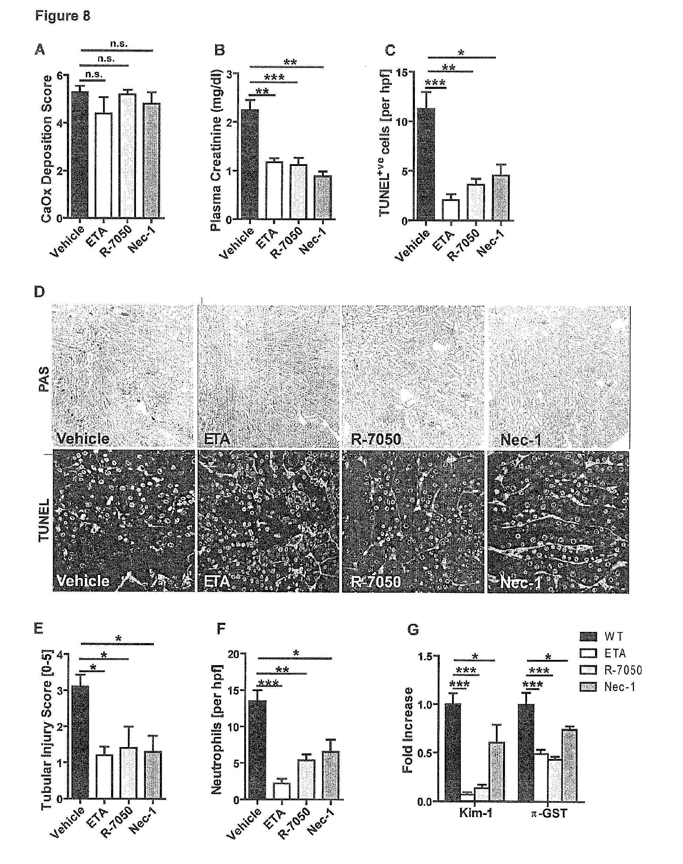

If TNFR/RIPK1/RIPK3/MLKL-mediated necroptosis drives tubule necrosis in CN then blockade of either TNFα, TNFR or RIPK1 might be a valuable therapeutic strategy. To test this concept mice were treated with the TNFα blocker etanercept, the TNFR blocker R-7050 or with the RIPK1 antagonist necrostatin-1. Oxalate exposure resulted in equal amounts of CaOx crystal deposits in all groups of mice but etanercept, R-7050, and necrostatin-1 all significantly reduced all functional and structural aspects of CN, i.e. serum creatinine levels, tubule necrosis, neutrophil influx, and the mRNA expression of kidney injury markers (Figure 8). Thus, crystal-induced TNFα release and the subsequent TNFR-triggered activation of RIPK1-RIPK3-MLKL-executed tubule necroptosis represent therapeutic targets in CN and potentially other crystallopathies, for example, using etanercept, R-7050 or necrostatin-1 as respective antagonistic drugs.

More specifically, figures 1-12show the following:
**Figure 1** **shows that crystals induce primary cell necrosis.** A: Various crystals were incubated with tubular epithelial cells as indicated. Images show the crystal shapes at a magnification of 1000x (left) and how they were loaded on monolayers of tubular epithelial cells (middle, 200x). The images on the right show the same rhodamin-labelled monolayers (red) 6 hours later. When Sytox green is added to the medium cells with permeable plasma membranes turn green indicating cell death (200x). B: Flow cytometry was used to define the type and stage of tubular cell (MTC) death upon calcium oxalate (CaOx) crystal exposure or ultraviolet light type B (UVB) for 90 seconds (s) over a period of 50 hours as described in methods. Data for viable cells, apoptotic cells, and necrotic cells are expressed as the percentage of viable cells ± SEM of all cells for each time point. C: The graphs show a quantitiative analysis of the same experiment displaying all different phenotypes of tubular epithelial cells. D: Tubular epithelial cell viability upon crystal exposure by MTT assay with and without the pan-caspase inhibitor ZVAD-FMK. All data are SEM±means of at least 3 independent experiments. Calcium oxalate (CaOx), monosodium urate (MSU), calcium pyrophosphate dehydrate (CPPD), N.S. = not significant. * p<0.05 versus medium control, *** p<0.001 versus respective control.
**Figure 2** **shows that TNFRs and RIPK-1 mediate crystal cytotoxicity.** A: Tubular epithelial cells were isolated from wild-type (WT) or *Tnfr1*/*2-* and *Nlrp3*-deficient mice and exposed to calcium oxalate (CaOx) crystals. Cell viability was assessed by MTT assay 24h later. Data are expressed as mean cell viability ± SEM of three independent experiments. Baseline viability is set as 100%. B:RT-PCR for TNFR1, RIPK1, RIPK3, and MLKL was performed on cell total RNA isolates taken 6h after stimulation of tubular epithelial cells exposed to CaOx crystals (300µg/ml). C: Western blot for TNFR1, RIPK1, RIPK3, and β-actin were performed on cell isolates from similar experiments after 24h. D: Tubular epithelial cells were exposed to different amounts of CaOx, monosodium urate (MSU), calcium pyrophosphate dehydrate (CPPD) or cystine crystals as indicated in the presence or absence of the RIPK1 inhibitor necrostatin-1 together with the pan-caspase-inhibitor zVAD-FMK. Cell viability was assessed by MTT assay 24h later. Data are expressed as mean cell viability ± SEM of three independent experiments. Baseline viability is set as 100%. E: In similar experiments necrotic tubular epithelial cells were identified via propidium iodine positivity and the results were expressed as mean fluorescent intensity on digital analysis of pictures taken from culture dishes. Representative images are shown at an original magnification of 200x. * p<0.05, ** p<0.01, *** p<0.001 versus respective medium control. N.S. = not significant.
**Figure 3** **shows that lack or suppression of RIPK1, RIPK3, and MLKL prevents crystal cytotoxicity.** A: Tubular epithelial cells were transfected with specific small inhibitor (si) RNA for RIPK1, RIPK3, MLKL or a control siRNA of scrambled sequence before being exposed to calcium oxalate (CaOx) crystals. Cell viability was assessed by MTT assay 24h later. B: Primary tubular cells were isolated from WT, *Ripk3-* and *Mlkl*-deficient mice and were exposed to CaOx crystals. Cell viability was measured at different time points by MTT assay. C: Tubular epithelial cells were pretreated with RIPK3 inhibitor at different concentrations before exposed to CaOx, MSU, CPPD and cystine. Cell viability was measured by MTT assay 24hr later. Data are expressed as mean cell viability ± SEM of three independent experiments. Baseline viability is set as 100%. * p<0.05, ** p<0.01, *** p<0.001 either versus control siRNA or wild type mice, respectively.
**Figure 4** **shows that acute oxalate nephropathy involves tubular epithelial cell necrosis.** Oxalate exposure leads to diffuse intrarenal crystal formation as displayed by Pizzolato stain (A). In contrast to healthy control mice (B) CaOx crystals form large stone-like plugs that obstruct the intratubular lumen of some tubules (C) Smaller crystals attach to the luminal membrane and also appear within intracellular compartments of the tubular cells (C). D: Scanning electron microscopy allowing a view into the lumen of such affected tubules confirms the direct interaction of crystals with the luminal membrane of the cells. Transmission electron microscopy shows intact proximal (E) and distal tubules (F) of healthy control mice and luminal crystal plugs and intracellular crystals in mice upon CaOx exposure (G). In many tubules crystal deposition is spatially associated with signs of tubular epithelial cell necrosis, which becomes evident from organelle swelling, cytoplasma translucence, and rupture of the plasma membrane (H and I). J: Freeze fracture electron microscopy also shows plasma membrane rupture of tubular epithelial cells bringing CaOx crystals in direct contact with the cell cytoplasma. Three representative images of this process are shown.
**Figure 5** **shows that oxalate nephropathy in C57BL/6 mice requires RIPK3 and MLKL.** A: Oxalate feeding to C57BL/6 mice (n=5) induced intrarenal mRNA expression of TNFR1, RIPK1, RIPK3, and MLKL as determined by RT-PCR from total kidney isolates. B: Oxalate feeding to wild-type mice as well as to *Ripk3-* and *Mlkl*-deficient mice of the same genetic background (n=5 in each group) resulted in identical amounts of CaOx crystal deposition after 24h as quantified by morphometry of Pizzolato-stained kidney sections. Oxalate nephropathy in wild-type mice was associated with increased plasma creatinine (C) and BUN levels (D), which was both attenuated in the gene deficient mouse strains. E: PAS staining illustrated tubular necrosis at the corticomedullary junction in wild-type mice. Original image magnification: x100. TUNEL staining identified dying cells (red), with counterstaining for laminin (green) and cell nuclei (DAPI, white). Original image magnification: x200. F: Tubular injury was quantified by semiquantitative scoring of PAS-stained section as described in methods. G: Neutrophils were identified by immunostaining and counted per high power field (hpf). H: RT-PCR from total kidney isolates quantified intrarenal mRNA expression of the tubular injury markers Kim-1 and πGST. Data are means ± SEM from 5 mice in each group. * p<0.05, *** p<0.001 versus wild-type mice.
**Figure 6** **shows RIPK1 inhibition and neutrophil recruitment.** A-B: Intraperitoneal injection of calcium oxalate (CaOx), monosodium urate (MSU), calcium pyrophosphate dehydrate (CPPD) or cystine crystals into C57BL/6 mice (n=4 in each group) induces neutrophil recruitment to the peritoneal cavity as determined by flow cytometry of peritoneal lavage fluids. This process is not affected by concomitant necrostatin-1 treatment. A: shows representative flow cytometry data for each crystal presented as mean fluorescence intensity for the neutrophil marker. B: shows data of each mouse with the mean of wild-type mice set as 100%. N.S. = not significant. C: Similar experiments using the air-pouch model of neutrophil recruitment gave identical results (n=5 in each group) and are expressed in the same manner. D-F: In vivo microscopy studies of the postischemic musculus cremaster were performed as a second model of injury-associated and ROS-dependent neutrophil recruitment in C57BL/6 mice. Necrostatin-1 significantly reduced the microvascular leakage of FITC-labelled dextran particles as illustrated by representative images in D at an original magnification of 400x and quantitatively in E. F: In the same experiment leukocyte transmigration from the microvasculature was quantified by counting from video recordings taken at baseline and at 60 and 120 minutes. Data are means ± SEM from 7 mice in each group. * p<0.05, * p<0.01, *** p<0.001 versus vehicle control.
**Figure 7** **shows that oxalate nephropathy in C57BL/6 mice requires TNFR1.** A: Oxalate feeding to wild-type mice as well as to *Tnfr1-* and *Tnfr1*/2-deficient mice of the same genetic background (n=5 in each group) resulted in identical amounts of CaOx crystal deposition after 24h as quantified by morphometry of Pizzolato-stained kidney sections. B: Oxalate nephropathy in wild-type mice was associated with increased plasma creatinine levels, which were attenuated to the same extend in both gene-deficient mouse strains. C: Dying cells were quantified by TUNEL staining. D: PAS staining illustrated tubular necrosis at the corticomedullary junction in wild-type mice. Original image magnification: x100. TUNEL staining identified dying cells (red), with counterstaining for laminin (green) and cell nuclei (DAPI, white). Original image magnification: x200. E: Tubular injury was quantified by semiquantitative scoring of PAS-stained section as described in methods. F: Neutrophils were identified by immunostaining and counted per high power field (hpf). G: RT-PCR from total kidney isolates quantified intrarenal mRNA expression of the tubular injury markers Kim-1 and πGST. Data are means ± SEM from 5 mice in each group. * p<0.05, *** p<0.001 versus wild-type mice.
**Figure 8** **shows that etanercept, R-7050, and necrostatin-1 abrogate crystal nephropathy.** A: Oxalate feeding to wild-type mice treated either with vehicle, the TNF-α blocker etanercept, the TNFR blocker R-7050 or the RIPK1 inhibitor necrostatin (Nec)-1 (n=5 in each group) resulted in identical amounts of CaOx crystal deposition after 24h as quantified by morphometry of Pizzolato-stained kidney sections but significantly reduced the levels of plasma creatinine (B) and the number of TUNEL positive cells in kidney sections (C). C: PAS staining illustrated tubular necrosis at the corticomedullary junction in wild-type mice. Original image magnification: x100. TUNEL staining identified dying cells (red), with counterstaining for laminin (green) and cell nuclei (DAPI, white). Original image magnification: x200. D and E: Tubular injury was quantified by semiquantitative scoring of PAS-stained section as described in methods. F: Neutrophils were identified by immunostaining and counted per high power field (hpf). G: RT-PCR from total kidney isolates quantified intrarenal mRNA expression of the tubular injury markers Kim-1 and πGST. Data are means ± SEM from 5 mice in each group. * p<0.05, *** p<0.001 versus vehicle-treated mice.
**Figure 9** **shows cell viabilities of tubular epithelial cells.**
   Tubular epithelial cells were exposed to either calcium oxalate (CaOx) crystals or 300ng/ml of recombinant TNF-α and cell viability was determined by MTT assay 24h later. Data are expressed as mean cell viability ± SEM of three independent experiments. Baseline viability is set as 100%. * p<0.05, *** p<0.001 either versus medium control.
**Figure 10** **shows cell viabilities of human dermal fibroblasts, L929 cells and HK-2 cells.**
   Human dermal fibroblasts, L929 cells, and HK-2 cells were all exposed to calcium oxalate (CaOx), monosodium urate (MSU), calcium pyrophosphate dehydrate (CPPD), and cystine crystals at different concentrations as indicated in the presence or absence of zVAD-FMK and necrostatin (Nec)-1. Cell viability was determined by MTT assay 24h later. Data are expressed as mean cell viability ± SEM of three independent experiments. Baseline viability is set as 100%. * p<0.05, ** <0.01 either versus vehicle control.
**Figure 11** **shows results of air pouch experiments.**
   Calcium oxalate (CaOx), monosodium urate (MSU), calcium pyrophosphate dehydrate (CPPD) or cysteine crystals were injected into air pouch of Balb/c mice in the presence or absence of necrostatin (Nec)-1. Data are cytokine ELISA results of air pouch fluids taken 24h after crystal injection. Data are means ± SEM of 4 mice in each group. * p<0.05 versus vehicle control. N.S. = not significant.
**Figure 12** **shows the effects of oxalate exposure.**
   Wild-type C57BL/6 mouse kidney mRNA expression of TNF-α (A), TNFR1 (B), and TNFR2 (C) of 24h after oxalate exposure. Respective western blots are shown below using β-actin as loading control. D: Flow cytometry for intracellular TNF-α of kidney cell suspensions from the same experiment identifies various leukocytes subsets as well as CD45- renal parenchymal cells as a source of TNF-α protein expression. Quantitative data are means ± SEM of 5 mice in each group.

### Example 3

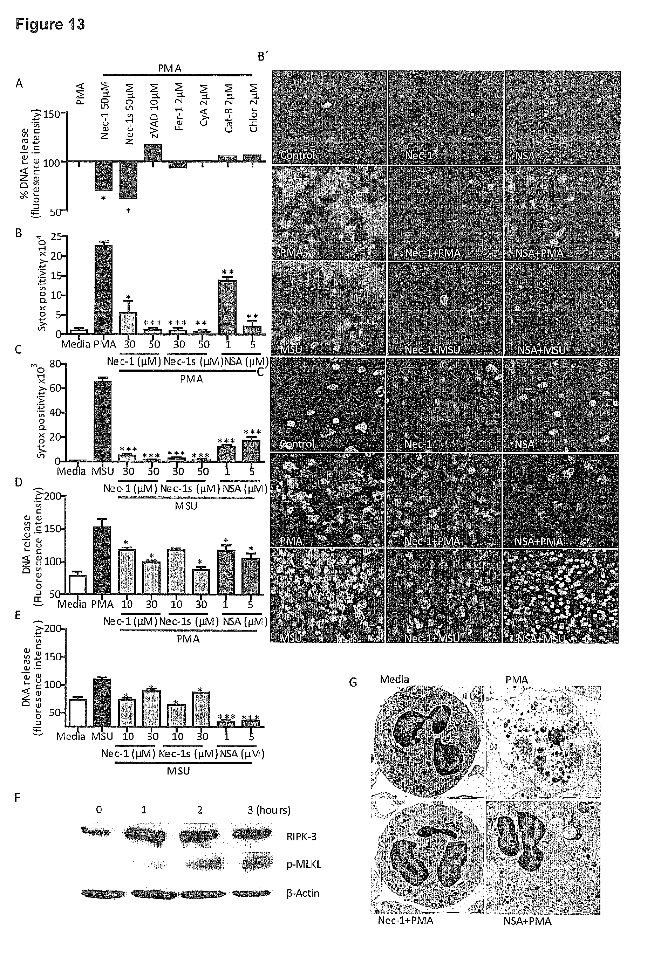

### Figures 13-18 show the role of RIPK1 and RIPK3 in NET formation.

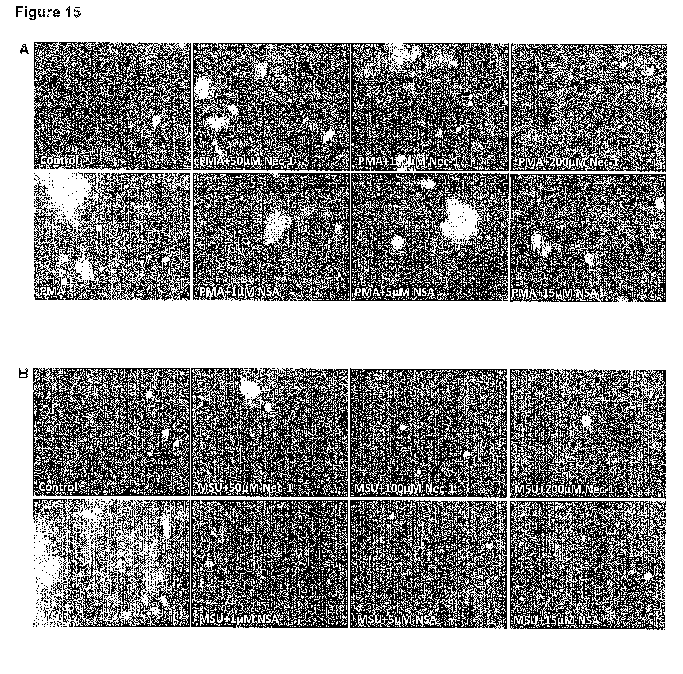

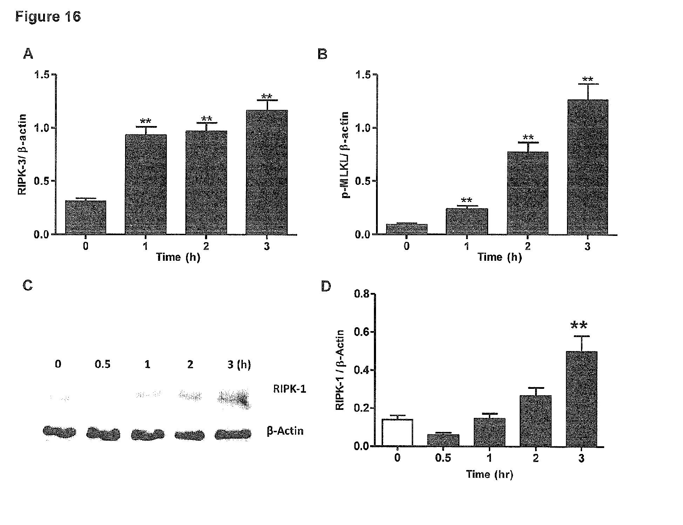

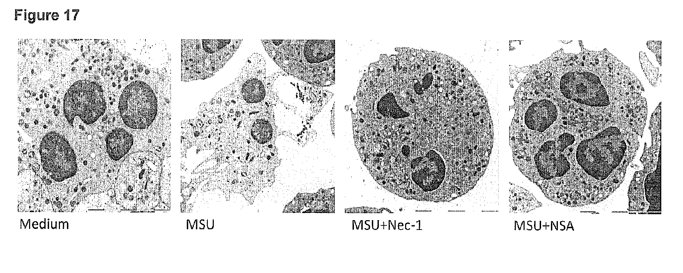

First, several cell death pathway inhibitors were tested in human neutrophils undergoing PMA-induced NET formation. Neither zVAD-FMK, ferrostatin-1 (Fer-1) or cyclosporine A affected NET formation (Figure 13A), which excluded caspase-mediated extrinsic apoptosis or pyroptosis, glutathione peroxidase-4-mediated ferroptosis or cyclophilin D-mediated mitochondrial permeability transition respectively. In contrast, first generation Nec-1 as well as Nec-1s, selective inhibitors of RIPK1 phosphorylation suppressed nuclear DNA dye uptake, chromatin decondensation, NET formation, and DNA release in human neutrophils upon exposure of MSU crystals and PMA (Figure 13B-E and figure 15). Outside-in signaling trigged RIPK1 activation is a critical step in TLR- or death receptor-mediated necroptosis. Activated RIPK1 is thought to phosphorylate RIPK3 to form the necrosome, which subsequently recruits and phosphorylates Mixed Lineage Kinase domain-Like (MLKL) subsequently executing plasma membrane rupture. PMA-induced NET formation of human neutrophils was associated with induction of RIPK1, RIPK3, and phosphorylation of MLKL (Figure 13F and figure 16). In fact, blocking MLKL oligomerization and cell death with necrosulfonamide abrogated MSU crystal- and PMA-induced NET formation in human neutrophils (Figure 13B-D and figure 15). Transmission electron microscopy displayed that Nec-1 or necrosulfonamide inhibited the rupture of nuclear membrane and plasma membrane of neutrophils upon for PMA or MSU stimulation (Figure 13F and figure 17). These findings document that activation of RIPK1, RIPK3, and pMLKL precedes and is required for trigger-induced NET formation of human neutrophils. G: Transmission electron microscopy of human neutrophils stimulated with PMA, which induces neutrophil disintegration. Note that Necrostatin (Nec)-1 and necrosulfonamide (NSA) prevents this process.

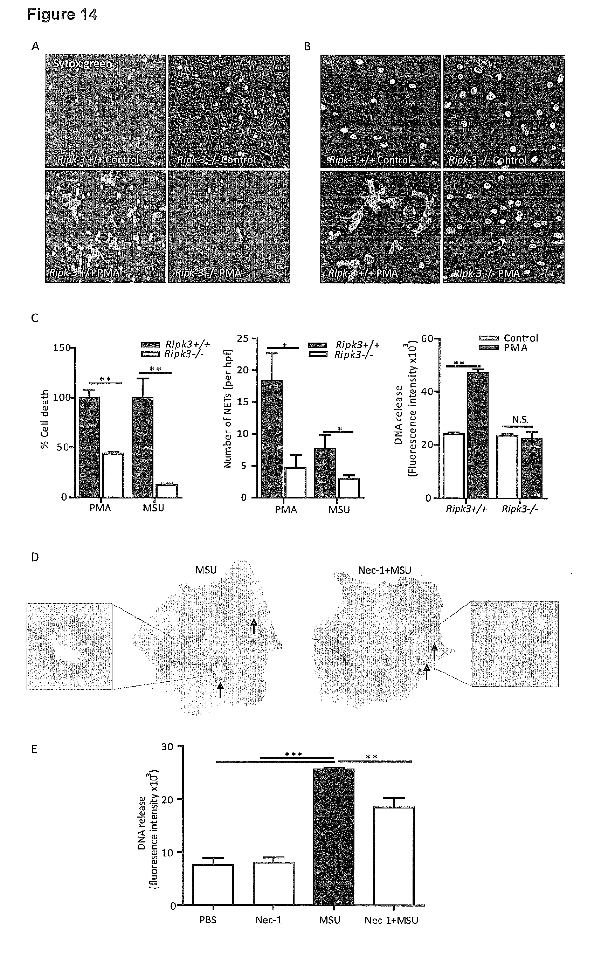

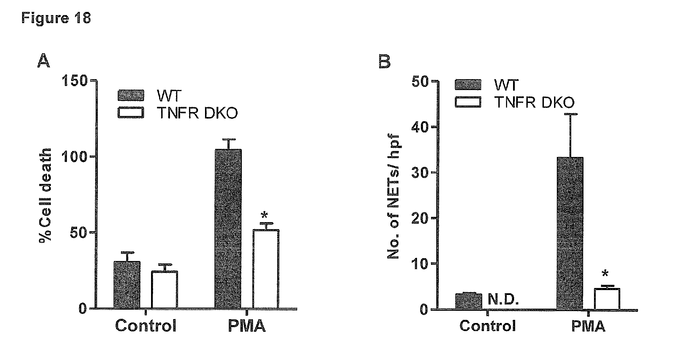

To provide functional data also on RIPK3, neutrophils from wild-type and Ripk3-deficient mice on identical genetic backgrounds were isolated and exposed to PMA or MSU to induce NET formation. Ripk3-deficient neutrophils completely lacked nuclear DNA dye uptake, chromatin decondensation, NET formation, and DNA release as compared to Ripk3^{+/+} wild type cells (Figure 14A-C). These data confirm the role of RIPK3 in NET formation. Activated neutrophils release TNF-alpha, which could trigger necroptosis in an autocrine manner. In fact, neutrophils from Tnfr1/Tnfr2-double-deficient mice were partially protected from PMA-induced cell death and NET formation imposing a role for this autocrine pathway (figure 18). To validate these in vitro results 2.5 mg MSU crystals were injected into subcutaneous air pouches at the back of Balb/c mice and the effects of RIPK1 inhibition with 1.65 mg/kg Nec-1 on MSU-induced crystal-NET conglomerates, i.e. tophus formation, were analyzed. Nec-1 had no effect on neutrophil recruitment into the air pouch per se but significantly reduced tophus mass along the pouch membrane as determined by visual inspection (Figure 14D). In addition, Nec-1 significantly reduced NET-related DNA release into the lavage fluid (Figure 14E).

Nuclear desegmentation, chromatin decondensation, and NET formation are unique features in cell death research, but our data now provide evidence that NET formation involves the RIPK1-, RIPK3- and MLKL-dependent signaling pathway, which implies that "NETosis" rather represents "neutrophil necroptosis". Necroptosis is a well-defined category of regulated necrosis following activation of the death receptors TNFR1, CD95, and TRAILR, Toll-like receptor-3/-4, interferon-γ receptor, and presumably the T cell receptor. Phosphorylated MLKL is critical for necroptosis and was shown to localize to phophatidylinositol triphosphates at the inner aspect of the plasma membrane where it triggers membrane rupture by undefined mechanisms. It appears likely that the rupture of the neutrophil's nuclear membrane, mitochondrial membranes and plasma membrane preceding NET formation also involve this mechanism. pMLKL probably not only binds to the plasma membrane, but also to organelle and nuclear membranes.

Together, RIPK1, RIPK3, and MLKL represent molecular targets to inhibit NET formation for example with necrostatins and necrosulfonamide. This has important implications for the prevention or treatment of NET-related disorders.

More specifically figures 13-18 show the following:
**Figure 13** shows human neutrophil studies. A: Human neutrophils were exposed to PMA in the presence of several cell death inhibitors Nec-1 (50µM), Nec-1s (50µM), zVAD-FMK (10µM), Ferrostatin-1 (Fer-1, 2µM), cyclosporine A (CyA, 2µM), cathepsin-B inhibitor (Cat-B, 2µM), chloroquine (Chlor, 2µM) as indicated. DNA release into the supernatant was quantified by PicoGreen detection after 2 hours as a marker of NET-related chromatin release. PMA was set as 100%. B, C and B': Sytox green immunofluorescence 2 hours after PMA or monosodium urate (MSU) crystal exposure to human neutrophils detects permeability of plasma and nuclear membranes. Representative images are shown at an original magnification of 200x. C': Confocal microscopy for same cells stained with FITC-anti-histones (green) and TOPRO (blue) to detect histones and DNA in extracellular traps. Representative images are shown at an original magnification of 400x. A quantitative analysis of Sytox+ death cells and NETs is shown in the bar graphs where data are expressed as means ± standard error of the mean. D and E: DNA release into the supernatant was quantified by PicoGreen detection as a marker of NET-related chromatin release after 2 hours of PMA (D) and MSU stimulation (E). Data are expressed as means ± standard error of the mean. F: Western blot for RIPK3 and phosphorylated MLKL at different time points after PMA stimulation of human neutrophils. β-actin serves as loading control. G: Transmission electron microscopy images of human neutrophils exposed to PMA in the presence or absence of necrosulfonamide (NSA). *p<0.05, **p<0.01 versus medium control.
**Figure 14** shows mouse neutrophil studies. Neutrophils were isolated from the blood of wild type and Ripk3-deficient mice and exposed to PMA. Sytox green (A) and confocal microscopy (B) identify dying cells and NETs, respectively. C: DNA release into the supernatant of the same experiment detected by PicoGreen. Data in all graphs are means ± standard error of the mean. D: Air pouch membranes of mice 24h after injection of 2.5mg of MSU crystals. Note the tophus-like mass (arrows), which is largely absent upon co-injection of necrostatin-1. E: DNA release into the pouch lavage fluid of the same experiment detected by PicoGreen. Data in all graphs are means ± standard error of the mean. **p<0.01, ***p<0.001 versus medium control. N.S = not significant.
**Figure 15** shows sytox staining of human neutrophils after 3h of exposure to various stimuli and necrostatin-1 (Nec-1) and necrosulfonamide (NSA) as indicated. Representative images are shown at a magnification of 200x in panels A and B.
**Figure 16** shows quantitative analysis of Western blots for RIPK3 (A), p-MLKL (B), and RIPK1 (C and D) at different time points after PMA stimulation of human neutrophils. ** p<0.01, *** p<0.001 versus baseline.
**Figure 17** shows transmission electron microscopy images of human neutrophils exposed to monosodium urate (MSU) crystals in the presence or absence of necrostatin-1 (Nec-1) or necrosulfonamide (NSA).
**Figure 18** shows neutrophils were isolated from wild type (WT) and Tnfr1/2-double deficient (double knock-out/DKO) mice which were exposed to PMA. Cell death was evaluated by Sytox staining (A) and NET formation by confocal microscopy (B). Data are means ± standard error of the mean. *p<0.05 versus wild type. N.D = not detected.

The inventors had hypothesized that NET formation involves one of the previously identified signaling pathways of regulated necrosis. The data above excluded caspase-dependent apoptosis and pyroptosis, and ferroptosis, but rather identified that NET formation depends on RIPK1, RIPK3, and MLKL. As this pathway defines the cell death category of "necroptosis", the process of neutrophil death leading to NET formation, previously named *"NETosis",* rather represents "neutrophil necroptosis".

Nuclear desegmentation, chromatin decondensation, and NET formation are unique features in cell death research, but the above data now provide evidence that NET formation involves the RIPK1-, RIPK3- and MLKL-dependent signaling pathway. As the current classification of cell death no longer relies on morphological criteria but rather on specific biochemical and signalling pathway criteria the above data imply that cell death associated with NET formation, currently referred to as "NETosis" should be termed "neutrophil necroptosis", in accordance with a definition of necroptosis as a RIPK3-dependent necrotic type cell death. Necroptosis is a well-defined category of regulated necrosis following activation of the death receptors TNFR1, CD95, and TRAILR, Toll-like receptor-3/-4, interferon-γ receptor, and presumably the T cell receptor. This includes several of the known triggers of NET formation, while the processes leading to PMA- and MSU-induced RIPK1/RIPK3/MLKL activation remain to be identified. But how does MLKL phosphorylation lead to NET formation? Phosphorylated MLKL is critical for necroptosis and was shown to localize to phophatidylisonitol triphosphates at the inner aspect of the plasma membrane where it triggers membrane rupture by undefined mechanisms. It seems likely that the rupture of the neutrophils nuclear membrane, mitochondrial membranes and plasma membrane preceding NET formation also involve this mechanism, although this remains to be demonstrated. In this regard, it is worth mentioning that pMLKL probably not only binds to the plasma membrane, but also to organelle and nuclear membranes. Importantly, a RIPK3 effect on other proteins may modulate the sensitivity to necrosis, but the dependency on MLKL suggests that a non-MLKL-dependent pathway cannot cause cell death in this pathway.

As a second conclusion RIPK1, RIPK3, and MLKL represent valid molecular targets to inhibit NET formation for example with necrostatins and necrosulfonamide. This has important implications for the prevention or treatment of NET-related disorders such as any form of vascular thrombosis, atherosclerosis, myocardial infarction, sepsis, gout and other crystallopathies, or autoimmune vasculitis.

Together, these data show that NET formation is a consequence of RIPK1/RIPK3, and MLKL-dependent neutrophil necroptosis, and that RIPK1, RIPK3 or MLKL represent molecular targets to prevent NET-related tissue injury.

The features of the present invention disclosed in the specification, the claims, and/or in the accompanying drawings may, both separately and in any combination thereof, be material for realizing the invention in various forms thereof.

## Claims

1. An inhibitor of the receptor-interacting serine/threonine-protein kinase 1 (RIPK1)-dependent necrosis pathway for use in a method of treatment of a crystallopathy or a neutrophil extracellular trap (NET)-formation-associated disease.

2. The inhibitor for use according to claim 1, wherein the inhibitor of the RIPK1-dependent necrosis pathway inhibits said pathway by inhibiting any of the following:
- binding of TNFα to TNFα-receptor (TNFR),
- internalization of TNF receptor into a cell,
- activity or expression of receptor-interacting serine/threonine-protein kinase 1 (RIPK1),
- activity or expression of receptor-interacting serine/threonine-protein kinase 3 (RIPK3),
- activity or expression of mixed lineage kinase-domain-like protein (MLKL),
or any combination of the foregoing

3. The inhibitor for use according to any of claims 1-2, wherein said inhibitor is
- an anti-TNFα-compound selected from etanercept, infliximab, adalimumab, inhibitors of TNF-receptor-internalization such as R7050, and anti-TNFα-siRNA,
- a RIPK1-inhibitor selected from necrostatin 1, necrostatin 1s, and anti-RIPK1-siRNA,
- a RIPK3-inhibitor selected from anti-RIPK3 siRNA, or
- a MLKL-inhibitor, preferably selected from necrosulfonamide and anti-MLKL-sirRNA

4. The inhibitor for use according to any of claims 1-3, wherein said crystallopathy is a disease selected from gout, pseudogout, silicosis, asbestosis, oxalosis, e.g. nephrocalcinosis, urolithiasis or nephrolithiasis, gallstones, pancreatic duct stones, cystinosis, atherosclerosis, implant-associated joint damage(s) and crystal-induced rhabdomyolysis.

5. The inhibitor for use according to claim 4, wherein said crystallopathy is selected from gout and oxalosis.

6. The inhibitor for use according to any of claims 1-4, wherein said neutrophil extracellular trap (NET)-formation-associated disease is selected from sepsis, thrombosis, autoimmune vasculitis, atherosclerosis, and myocardial infarction.

7. The inhibitor for use according to claim 6, wherein said NET-formulation-associated disease is selected from sepsis, thrombosis, atherosclerosis and myocardial infarction.

8. A pharmaceutical composition comprising the inhibitor according to any of claims 1-7 and a pharmaceutical excipient, for use in a method of treatment of a crystallopathy or a neutrophil extracellular trap (NET)-formation-associated disease, wherein said inhibitor, said crystallopathy and said NET-formation-associated disease are as defined in any of claims 1-7.

9. The inhibitor for use according to any of claims 1-7 or the pharmaceutical composition for use according to claim 8, wherein said treatment comprises administering a suitable amount of said inhibitor to a patient suffering from a crystallopathy or a NET-formation-associated disease.

10. Use of receptor-interacting serine/threonine-protein kinase 1 (RIPK1), receptor-interacting serine/threonine-protein kinase 3 (RIPK3) and/or of mixed lineage kinase domain-like protein in a screening method for identifying a compound useful for the treatment of a crystallopathy or a neutrophil extracellular trap (NET)-formation-associated disease.

11. Use according to claim 10, wherein said screening method for identifying involves:
- providing a candidate compound suspected of or to be tested for having an inhibitory action on RIPK1, RIPK3 or MLKL, and providing a known inhibitor of RIPK1, RIPK3, or MLKL,
- determining the action of said candidate compound and of a known inhibitor of RIPK1, RIPK3 or MLKL, on RIPK1, RIPK3 or MLKL,
- comparing the determined action of said candidate compound with the determined action of said known inhibitor of RIPK1, RIPK3 or MLKL, and
- identifying said candidate compound as being useful for the treatment of a crystallopathy or NET-formation-associated disease, if the action of said candidate compound on RIPK1, RIPK3 or MLKL is the same as the action of said known inhibitor, or if the action of said candidate compound on RIPK1, RIPK3 or MLKL is not less than 50% of the action of said known inhibitor, preferably not less than 40%, more preferably not less than 30%, even more preferably not less than 20%, and most preferably not less than 10% of the action of said known inhibitor.

12. A method of treatment of a crystallopathy or a neutrophil extracellular trap (NET)-formation-associated disease, comprising administering a suitable amount of an inhibitor of the RIPK1-dependent necrosis pathway, said inhibitor being as defined in any of claims 1-7, or of a pharmaceutical composition, as defined in claim 8, to a patient suffering from a crystallopathy or a NET-formation-associated disease.

13. The method for treatment according to claim 12, wherein said crystallopathy and said NET-formation-associated disease are as defined in any of claims 4-7.

14. The inhibitor or pharmaceutical composition for use according to claim 9 or the method of treatment according to any of claims 12-13, wherein said suitable amount is an amount in a range of from 0.001 mg/kg body weight of said patient to 1 g/kg body weight of said patient.

15. The inhibitor or pharmaceutical composition for use according to any of claims 9 and 14 or the method of treatment according to any of claims 12-14, wherein said patient is human.
